# EUROPÄISCHE PATENTSCHRIFT

(11) **EP 1 311 517 B1**
(45) Veröffentlichungstag und Bekanntmachung des Hinweises auf die Patenterteilung: **14.07.2004**
(21) Anmeldenummer: 01971921.0
(22) Anmeldetag: 17.08.2001
(51) Int. Cl.: C07F 9/46, C07F 9/48, C07F 9/50

(54) **CYCLOALIPHATISCH-AROMATISCHE DIPHOSPHINE UND IHRE VERWENDUNG IN DER KATALYSE**
CYCLOALIPHATIC/AROMATIC DIPHOSPHINES AND USE THEREOF IN CATALYSIS
DIPHOSPHINES CYCLOALIPHATIQUES/AROMATIQUES ET LEUR UTILISATION EN CATALYSE

(30) Priorität: 17.08.2000 DE 10040726
(43) Veröffentlichungstag der Anmeldung: 21.05.2003
(73) Patentinhaber: Degussa AG, 40474 Düsseldorf (DE)
(72) Erfinder: BOSCH, Boris, E., 50668 Köln (DE); MONSEES, Axel, 60487 Frankfurt (DE); DINGERDISSEN, Uwe, 64342 Seeheim-Jugenheim (DE); KNOCHEL, Paul, 81475 München (DE); HUPE, Eike, 82061 Neuried (DE)
(86) Internationale Anmeldenummer: PCT/EP2001/009481
(87) Internationale Veröffentlichungsnummer: WO 2002/014330

(56) Entgegenhaltungen:
- HUPE, E.; KNOCHEL, P.: "Stereoselective Synthesis of Secondary Organuzinc Reagents and Their Reaction with Heteroaromatic Electrophiles" ORGANIC LETTERS, Bd. 3, Nr. 1, 2001, Seiten 127-130, XP002185951
- BENINCORI, T.; ET AL.: "2,2',5,5'-Tetramethyl-4,4'-bis(diphenylph osphino)-3,3'-bithiophene: A New, Very Efficient, Easily Accessible, Chiral Biheteroaromatic Ligand for Homoheneous Stereoselective Catalysis" J. ORG. CHEM., Bd. 65, Nr. 7, 2000, Seiten 2043-2047, XP002185952
- ZHU, G.; ET AL.: "Highly Enantioselective Rh-Catalyzed Hydrogenations with a New Chiral 1,4-Bisphosphine Containing a Cyclic Backbone" J. AM. CHEM. SOC., Bd. 119, Nr. 7, 1997, Seiten 1799-1800, XP002185953
- CEREGHETTI, M.; ET AL.: "(R)- and (S)-6,6'-Dimethyl- and 6,6'-Dimethoxy-2,2'-diiodo-1,1'-biphenyls: Versatile Intermediates for the Synthesis of Atropisomeric Diphosphine Ligands" TETRAHEDRON LETTERS, Bd. 37, Nr. 30, 1996, Seiten 5347-5250, XP004029500
- BOUDIER, A.; KNOCHEL, P.: "Palladium Catalyzed Stereoselective Cross-Couplings and Acylations of Chiral Secondary Diorganozincs" TETRAHEDRON LETTERS, Bd. 40, 1999, Seiten 687-690, XP004151418

## Beschreibung

Die vorliegende Erfindung beinhaltet neue unsymmetrische chirale Diphosphine eines gemischten aliphatisch-aromatischen Typs und Prozesse für deren Synthese, Komplexe dieser Verbindungen mit, sowie deren Verwendung als Katalysatoren.

Trisubstituierte Organophosphorverbindungen haben große Bedeutung als Liganden in der homogenen Katalyse. Durch Variation der Substituenten am Phosphor in solchen Verbindungen lassen sich die elektronischen und sterischen Eigenschaften des Phosphorliganden gezielt beeinflussen, so daß Selektivität und Aktivität bei homogen-katalytischen Prozessen gesteuert werden können.
Enantiomerenangereicherte chirale Liganden werden in der asymmetrischen Synthese bzw. asymmetrischen Katalyse eingesetzt, hier kommt es wesentlich darauf an, daß die elektronischen *und* die stereochemischen Eigenschaften des Liganden auf das jeweilige Katalyseproblem optimal abgestimmt sind. Es besteht ein großer Bedarf an chiralen Liganden, die sich stereochemisch oder/und elektronisch unterscheiden, um den für eine bestimmte asymmetrische Katalyse optimalen 'maßgeschneiderten' Liganden aufzufinden. Im Idealfall hat man daher ein vielseitig modifizierbares, chirales Ligand-Grundgerüst zur Verfügung, das sich in bezug auf seine sterischen und elektronischen Eigenschaften in breitem Rahmen variieren läßt.

Aus den Stand der Technik sind Diphosphine bekannt, die entweder zwei aromatische Ringe (Benincori, T.; et al; J. Org. Chem 65(7), 2000, 2043-2047; Cereghetti, M.; et al.; Tetrahedron Letters 37(30), 1996, 5347-5350) oder zwei aliphatische Ringe (Zhu, G; et al; J. Am. Chem. Soc. 119(7), 1997, 1799-1800) aufweisen.

Die Strukturvielfalt der bisher bekannten Phosphorliganden ist sehr groß. Die Gliederung dieser Liganden kann beispielsweise nach Stoffklassen erfolgen, und Beispiele für solche Stoffklassen sind Trialkyl- und Triarylphosphine, Phosphite, Phosphinite, Phosphonite, Aminophosphane usw. Diese Einteilung nach Stoffklassen ist insbesondere nützlich, um die elektronischen Eigenschaften der Liganden zu beschreiben.

Darüber hinaus ist eine Klassifizierung von Phosphorliganden nach ihren Symmetrieeigenschaften oder nach der Zähnigkeit der Liganden möglich. Diese Strukturierung trägt insbesondere der Stabilität, Aktivität und (potentiellen) Stereoselektivität von Metallkomplexen mit Phosphorliganden als Katalysatorvorstufen/Katalysatoren Rechnung. Neben den weit verbreiteten C₂-symmetrischen bidentaten Ligandsystemen wie DUPHOS, DIPAMP, BINAP oder DEGUPHOS rücken unsymmetrische bidentate Organophosphorliganden immer mehr in den Fokus in der asymmetrischen Katalyse. Wichtige Beispiele sind die große Klasse der vielseitig einsetzbaren chiralen Ferrocenylphosphinliganden wie z.B. JOSIPHOS, DPPM, die Bisphosphinitliganden wie CARBOPHOS, die besonders in der asymmetrischen Hydrierung von Olefinen und Iminen erfolgreich eingesetzt werden, oder die Phosphin-Phosphit-Liganden wie BINAPHOS oder BIPHEMPHOS, die Meilensteine in der asymmetrischen Hydroformylierung von Olefinen darstellen. Im folgenden sind einige Strukturen von Phosphor-Liganden gezeigt, die bereits in der asymmetrischen Katalyse erfolgreich eingesetzt wurden.

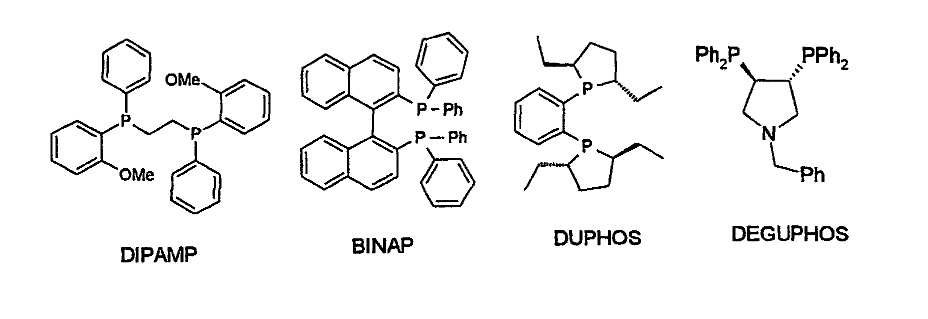

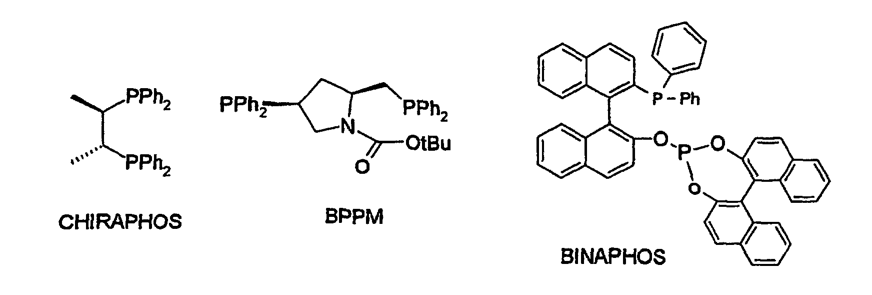

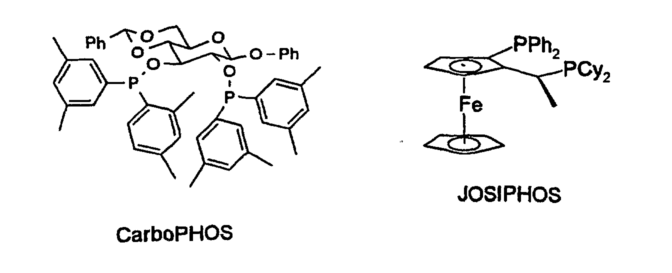

Ein wichtiger Aspekt des Erfolges dieser Verbindungsklassen wird der Schaffung einer besonders asymmetrischen Umgebung des Metallzentrums durch diese Ligandsysteme zugeschrieben. Um eine solche Umgebung für eine effektive Übertragung der Chiralität zu nutzen, ist es vorteilhaft, die Flexibilität des Ligandsystems als inhärente Limitierung der asymmetrischen Induktion zu kontrollieren.

Nachteilig bei den bisher bekannten chiralen Phosphorligandsystemen ist der relativ hohe präparative Aufwand bei Ihrer Darstellung zum einen und zum anderen relativ eingeschränkte Möglichkeiten der Variation der Eigenschaften eines gegebenen Ligandgrundgerüstes im Hinblick auf ein breites Anwendungsspektrum.

Aufgabe der vorliegenden Erfindung ist die Bereitstellung von neuartigen, unsymmetrischen, bidentaten und chiralen Phosphorligandsystemen, die die oben angeführten Merkmale für eine effektive asymmetrische Induktion in sich vereinigen, also eine hochasymmetrische Koordinationssphäre, mit unabhängig voneinander modifizierbaren Organophosphordonoren, schaffen, und die sich einfach in ihren sterischen und elektronischen Eigenschaften über einen außergewöhnlich weiten Raum modifizieren lassen.

Die Aufgabe wird durch chirale, unsymmetrische bidentate Organophosphorliganden der Formeln (Ia) und (Ib) gelöst. Das Grundgerüst der erfindungsgemäßen Verbindungen besteht aus jeweils einem chiralen cycloaliphatischen oder heterocycloaliphatischen und einem aromatischen oder heteroaromatischen Ringsystem, welche über eine direkte Kohlenstoff-Einfachbindung miteinander verknüpft sind. Die trivalenten Phosphorfunktionalitäten sind an diesem Grundgerüst jeweils in ortho-Position zu dieser Bindung an den beiden Ringsystemen angebunden.

Die vorliegende Erfindung betrifft somit Verbindungen der allgemeinen Formeln (Ia) und (Ib). 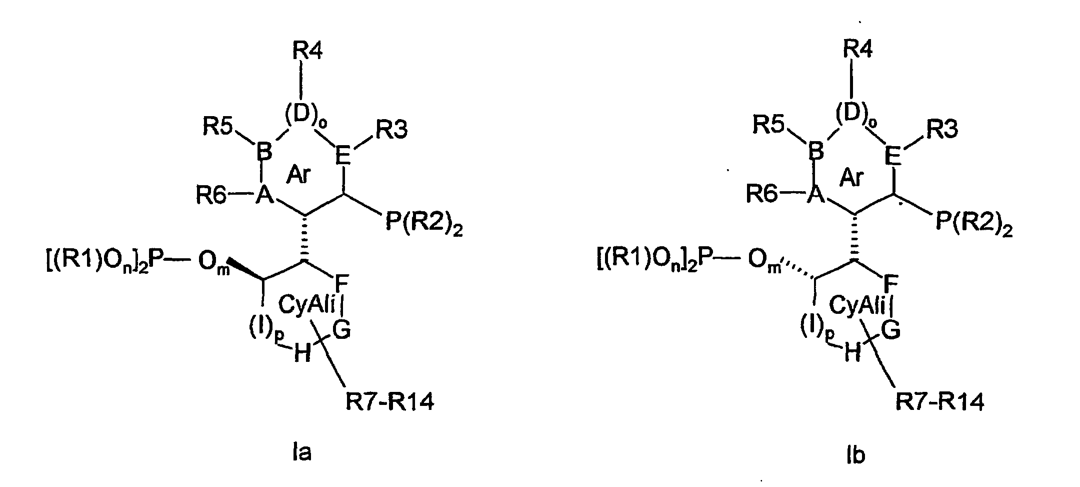 worin o und p unabhängig voneinander 0 oder 1 sein können und
- Ar: Teil eines sechsgliedrigen aromatischen oder 5-6 gliedrigen heteroaromatischen Ringsystems ist, wobei das heteroaromatische Ringsystem 1-3 Stickstoffatome, 1 Sauerstoffatom oder 1 Schwefelatom an den Positionen A, B, D und E enthalten kann.
Der sechsgliedrige Heteroaromat ist vorzugsweise ein Pyridyl-Rest, und der fünfgliedrigen Heteroaromat ist vorzugsweise ein Furyl, Thiophenyl oder Pyrrolyl.
- CyAli: ist Teil eines 5-6 gliedrigen cycloaliphatischen oder heterocycloaliphatischen Ringsystems ist, wobei das heterocycloaliphatische Ringsystem 1-2 Heteroatome aus der Gruppe N, O, S an den Positionen F, G, H und I enthalten kann.
Das Cycloaliphatische Ringsystem ist vorzugsweise ein Cyclohexyl- und Cyclopentyl- Rest oder ist als Teil eines annelierten Systems ein Indenyl- und Tetrahydronaphtalinyl- Rest. Das heterpaliphatische Ringsystem ist vorzugsweise ein Tetrahydro-furanyl-, Tetrahydrothiophenyl-, Pyrrolidinyl, Piperidinyl- Rest.
- R¹-R²,: unabhängig voneinander C₁-C₂₄Alkyl, C₃-C₈ Cycloalkyl, wobei der Cyclus auch 1-2 Heteroatome, ausgewählt aus der Gruppe N, O, S, enthahalten kann, C₆-C₁₄Aryl, Phenyl, Naphthyl, Fluorenyl, C₂-C₁₃ Heteroaryl, wobei die Zahl der Heteroatome, ausgewählt aus der Gruppe N, O, S, 1-4 betragen kann, wobei die cyclischen aliphatischen oder aromatischen Reste bevorzugt 5 bis 6 gliedrige Ringe sind; wobei die vorgenannten Gruppen selbst jeweils ein- oder mehrfach substituiert sein können, diese Substituenten können dabei unabhängig voneinander Wasserstoff, C₁-C₂₀ Alkyl, C₂-C₂₀ Alkenyl, C₁-C₁₀ Haloalkyl, C₃-C₈ Cycloalkyl, C₂-C₉ Heteroalkyl, C₆-C₈Aryl, Phenyl, Naphthyl, Fluorenyl, C₂-C₆ Heteroaryl, wobei die Zahl der Heteroatome, insbesondere aus der Gruppe N, O, S, 1-4 betragen kann, C₁-C₁₀ Alkoxy, bevorzugt OMe, C₁-C₉ Trihalomethylalkyl, bevorzugt Trifluormethyl und Trichlormethyl, Halogeno, besonders Fluoro und Chloro, Hydroxy, Trifluormethylsulfonato, Oxo, Thio, Thiolato, Amino, C₁-C₈ substituierte Amino der Formen NH₂, NH-Alkyl-C₁-C₈, NH-Aryl-C₅-C₆, N-Alkyl₂-C₁-C₈, N-Aryl₂-C₅-C₆, N-Alkyl₃-C₁-C₈⁺, N-Aryl₃-C₅-C₆⁺, NH-CO-Alkyl-C₁-C₈, NH-CO-Aryl-C₅-C₆, Cyano, Carboxylato der Formen COOH und COOQ wobei Q entweder ein einwertiges Kation oder C₁-C₈-Alkyl darstellt, C₁-C₆-Acyloxy, Sulfinato, Sulfonato der Formen SO₃H und SO₃Q wobei Q entweder ein einwertiges Kation, C₁-C₈-Alkyl oder C₆-Aryl darstellt, Phosphato der Formen PO₃H₂, PO₃HQ und PO₃Q₂ wobei Q entweder ein einwertiges Kation, C₁-C₈-Alkyl oder C₆-Aryl darstellt, Tri- C₁-C₆ Alkylsilyl, besonders SiMe₃, sein,
oder wobei R¹ mit dem Phosphoratom oder R² mit dem Phosphoratom einen 4-8 gliedrigen aliphatischen Zyklus bildet, der gegebenenfalls mit linearen oder verzweigten C₁-C₁₀ alkyl, C₆-aryl, Benzyl, C₁-C₁₀ Alkoxy, Hydroxy oder Benzyloxy, substituiert ist.
- R³-R¹⁴: unabhängig voneinander ein Wasserstoffatom oder C₁-C₂₄Alkyl, C₁-C₁₀-Haloalkyl, C₃-C₈ Cycloalkyl, C₃-C₈ Cycloalkenyl, wobei der Cyclus auch 1-2 Heteroatome enthalten kann, ausgewählt aus der Gruppe N, O, S, C₆-C₁₄ Aryl, Phenyl, Naphthyl, Fluorenyl, C₂-C₁₃ Heteroaryl, wobei die Zahl der Heteroatome, ausgewählt aus der Gruppe N, O, S, 1-4 betragen kann, wobei die cyclischen aliphatischen oder aromatischen Reste bevorzugt 5 bis 7 gliedrige Ringe sind; wobei die vorgenannten Gruppen selbst jeweils ein- oder mehrfach substituiert sein können, diese Substituenten können dabei unabhängig voneinander Wasserstoff, C₁-C₂₀ Alkyl, C₂-C₂₀ Alkenyl, C₁-C₁₀ Haloalkyl, C₃-C₈ Cycloalkyl, C₃-C₈ Cycloalkenyl, C₂-C₉ Heteroalkyl, C₁-C₉ Heteroalkenyl, C₆-C₈ Aryl, Phenyl, Naphthyl, Fluorenyl, C₂-C₆ Heteroaryl, wobei die Zahl der Heteroatome, insbesondere aus der Gruppe N, O, S, 1-4 betragen kann, C₁-C₁₀ Alkoxy, C₁-C₉ Trihalomethylalkyl, Trifluormethyl, Trichlormethyl, Fluoro, Chloro, Bromo, lodo, Hydroxy,
Trifluormethylsulfonato, Oxo, Thio, Thiolato, Amino, C₁-C₈ substituierte Amino der Formen NH₂, NH-Alkyl-C₁-C₈, NH-Aryl-C₅-C₆, N-Alkyl₂-C₁-C₈, N-Aryl₂-C₅-C₆, N-Alkyl₃-C₁-C₈⁺, N-Aryl₃-C₅-C₆⁺, NH-CO-Alkyl-C₁-C₈, NH-CO-Aryl-C₅-C₆, Cyano, Carboxylato der Formen COOH und COOQ wobei Q entweder ein einwertiges Kation oder C₁-C₈-Alkyl darstellt, C₁-C₆-Acyloxy, Sulfinato, Sulfonato der Formen SO₃H und SO₃Q wobei Q entweder ein einwertiges Kation, C₁-C₈-Alkyl oder C₆-Aryl darstellt, Phosphato der Formen PO₃H₂, PO₃HQ und PO₃Q₂ wobei Q entweder ein einwertiges Kation, C₁-C₈-Alkyl oder C₆-Aryl darstellt, Tri- C₁-C₆ Alkylsilyl, substituiert sein können,
und wobei zwei dieser Substituenten auch verbrückt sein können, bevorzugt so, daß jeweils benachbarte Substituenten so miteinander verbrückt sind, so daß eine 5-7 gliedrige cyclische aromatische oder aliphatische Verbindung vorliegt.
- m, n: sind unabhängig voneinander 1 oder 0 sind, und
- P: ist ein dreiwertiger Phosphor.

Die Erfindung betrifft ferner Komplexverbindungen, die ein derartiges, chirales Ligandsystem der Formeln (Ia) und (Ib)mit mindestens einem Metall enthalten.

Bevorzugt steht
- R¹-R²,: unabhängig voneinander für C₁-C₆ Alkyl, C₅-C₆ Cycloalkyl, C₆ Aryl, Phenyl, Naphthyl, C₄-C₅ Heteroaryl, wobei die Zahl der Heteroatome, ausgewählt aus der Gruppe N, O, S, 1 beträgt, wobei die vorgenannten aromatischen oder heteroaromatischen Gruppen selbst jeweils ein- bis dreifach substituiert sein können, diese Substituenten können dabei unabhängig voneinander Wasserstoff, C₁-C₆ Alkyl, C₂-C₄ Alkenyl, C₁-C₆ Haloalkyl, C₂-C₆ Heteroalkyl, C₆Aryl, Phenyl, Naphthyl, Fluorenyl, C₃-C₅ Heteroaryl, wobei die Zahl der Heteroatome aus der Gruppe N, O, S, 1-2 betragen kann, C₁-C₆ Alkoxy, bevorzugt OMe, C₁-C₉ Trihalomethylalkyl, bevorzugt Trifluormethyl und Trichlormethyl, Halogeno, besonders Fluoro und Chloro, Hydroxy, Trifluormethylsulfonato, Oxo, Amino, C₁-C₆ substituierte Amino der Formen NH₂, NH-Alkyl-C₁-C₆, NH-Aryl-C₆, N-Alkyl₂-C₁-C₆, N-Aryl₂-C₆, N-Alkyl₃-C₁-C₆⁺, N-Aryl₃-C₆⁺, NH-CO-Alkyl-C₁-C₆, NH-CO-Aryl-C₆, besonders NMe₂, NEt₂, Cyano, Carboxylato der Formen COOH und COOQ wobei Q entweder ein einwertiges Kation oder C₁-C₄-Alkyl darstellt, C₁-C₆-Acyloxy, Sulfinato, Sulfonato der Formen SO₃H und SO₃Q wobei Q entweder ein einwertiges Kation, C₁-C₄-Alkyl oder C₆-Aryl darstellt, Phosphate der Formen PO₃H₂, PO₃HQ und PO₃Q₂ wobei Q entweder ein einwertiges Kation, C₁-C₄-Alkyl oder C₆-Aryl darstellt, Tri- C₁-C₆ Alkylsilyl, besonders SiMe₃.

Vorteile dieser Liganden ist, daß sie ein hochassymmetrische Koordinationsphäre in einem Metallkomplex schaffen können. Über ihre einfache Modifizierbarkeit können die elektronischen und sterischen Eigenschaften der Liganden in weiten Bereichen variiert werden. So kann z. B. die Rigidität des Ligandenrückgrats über die Einführung unterschiedlicher Substituenten an den Grundstrukturen der Liganden verändert werden.

Vorzugsweise weist das erfindungsgemäße Ligandsystem in R¹-R¹⁴ Alkyl, Alkenyl, Cycloalkyl, Alkoxy, Trialkylsilyl oder/und Dialkylaminogruppen unabhängig voneinander auf, die jeweils 1 bis 20, insbesondere 1 bis 6 Kohlenstoffatome enthalten.

Aus der Gruppe der Alkyl- und Alkoxysubstituenten sind Methyl, Ethyl, n-Propyl, 1-Methylethyl, n-Butyl, 1-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, n-Heptyl, n-Octyl, n-Nonyl, n-Decyl, Methoxy, Ethoxy, 1-Propoxy, 2-Propxy, 1-Butoxy, 2-Butoxy, 1,1-Dimethyl-ethoxy bevorzugt.

Unter den cyclischen Alkylsubstituenten sind besonders bevorzugt substituierte und unsubstituierte Cyclopentyl-, Cyclohexyl-, Cycloheptyl- Reste.

Als Alkenylreste sind bevorzugte Reste Vinyl, Propenyl, Isopropenyl, 1-Butenyl, 2-Butenyl, 1-Pentenyl, 2-Pentenyl, 2-Methyl-1-butenyl, 2-Methyl-2-butenyl, 3-Methyl-1-butenyl, 1-Hexenyl, 1-Heptenyl, 2-Heptenyl, 1-Octenyl oder 2-Octenyl. Unter den cyclischen Alkenylsubstituenten sind besonders bevorzugt Cyclopentenyl, Cyclohexenyl, Cycloheptenyl und Norbomyl.

Unter Substituenten in R¹-R² sind besonders bevorzugt 1-Methylethyl, Cyclohexyl, Cyclopentyl, Phenyl, 2-Methyl-phenyl, 3,5-Dimethyl-phenyl, 4-Methyl-phenyl, 4-Methoxy-phenyl, 3,5-Bis-(trifluormethyl)-phenyl, 4-Trifluormethyl-phenyl, 3,5-Dimethyl-4-Methoxy-phenyl, 4-Phenoxyl, 4-Dialkyamino, 2-Alkylphenyl, 3-Alkylphenyl, 4-Alkylphenyl, 2,6-Dialkylphenyl, 3,5-Dialkylphenyl, 3,4,5-Trialkylphenyl, 2-Alkoxyphenyl, 3-Alkoxyphenyl, 4-Alkoxyphenyl, 2,6-Dialkoxylphenyl, 3,5-Dialkoxyphenyl, 3,4,5-Triialkoxyphenyl, 3,5-Dialkyl-4-Alkoxyphenyl, 3,5-Dialkyl-4-dialkylaminophenyl, 4-Dialkylamino, wobei die vorgenannten Alkyl- und Alkoxygruppen jeweils vorzugsweise 1 bis 6 Kohlenstoffatome enthalten, 3,5-Trifluormethyl, 4-Trifluormethyl, 2-Sulfonyl, 3-Sulfonyl, 4-Sulfonyl, ein bis vierfach halogenierte Phenyl und Naphtyl. Bevorzugte Halogensubstituenten sind F, Cl und Br.

Alle Haloalkyl- oder/und Haloarylgruppen weisen vorzugsweise die allgemeinen Formeln GHal₃, CH₂CHal₃, C₂Hal₅ auf, wobei Hal insbesondere für F, Cl und Br stehen kann. Besonders bevorzugt sind Haloalkyl- oder/und Haloarylgruppen der Formeln CF₃, CH₂CF₃, C₂F₅.

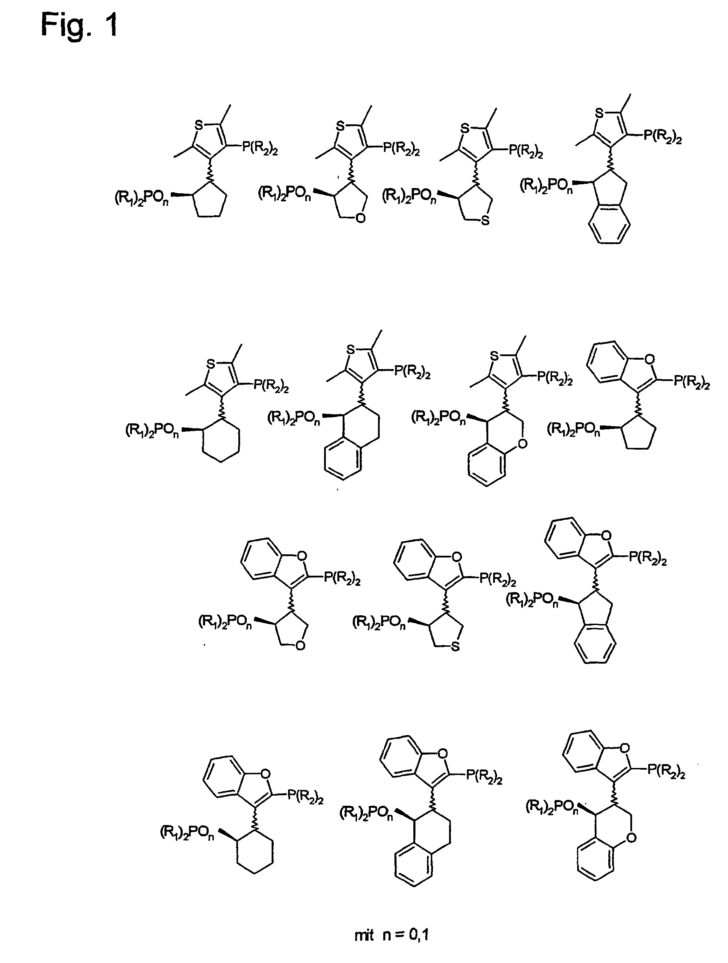

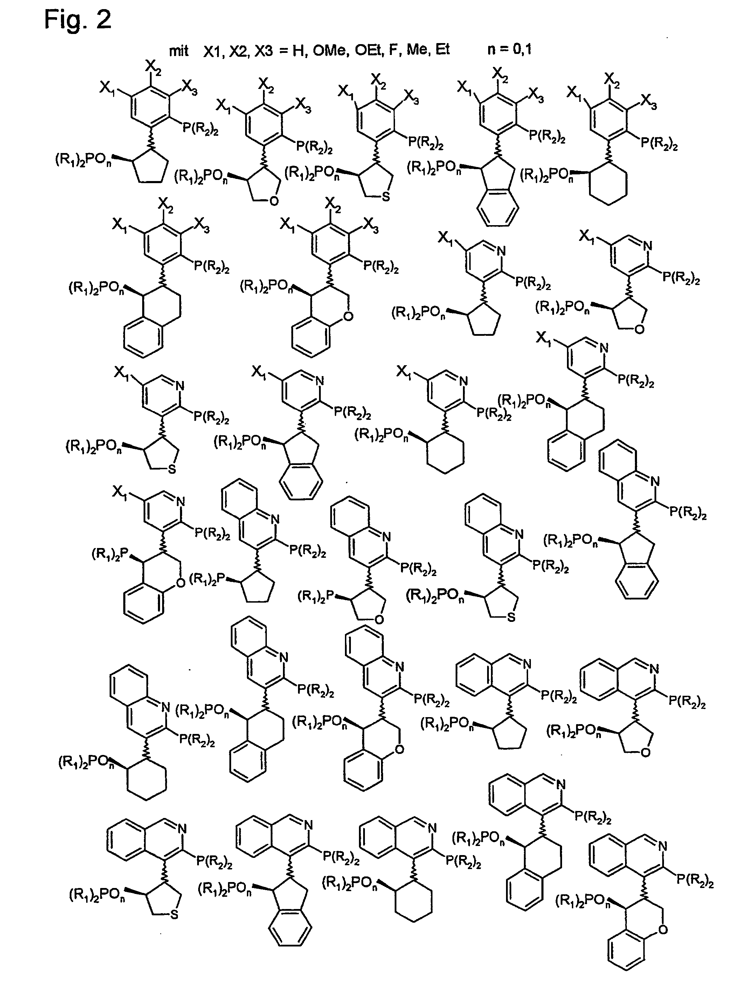

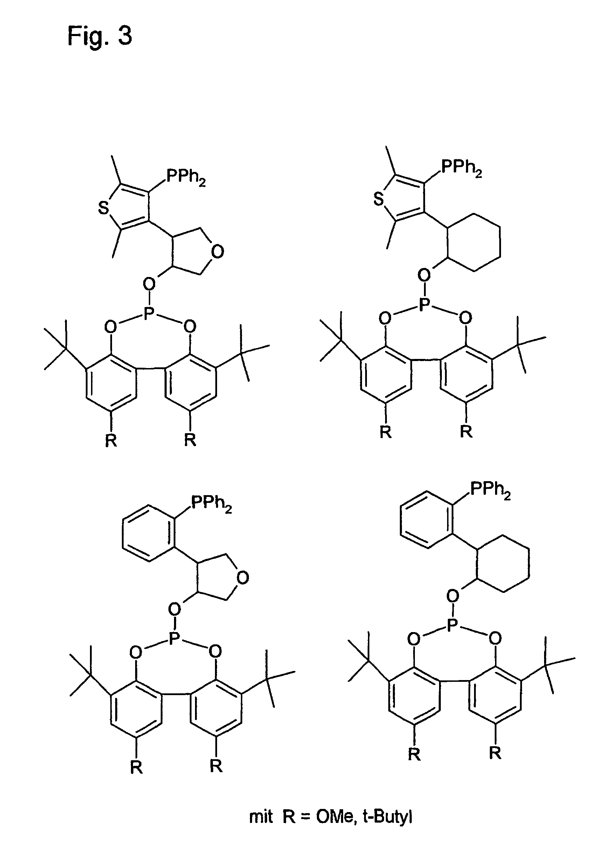

Schließlich sind Ligandsysteme der Formeln (Ia) und (Ib) als optisch aktive Ligandsysteme bevorzugt, bei denen ein Enantiomer angereichert ist. Besonders bevorzugt sind Ligandsysteme, bei denen die Enantiomerenanreicherung 90 %, insbesondere 99 % übersteigt. Besonders bevorzugte Ligandsysteme sind in Fig. 1, 2 und 3 abgebildet.

Diese bevorzugten Liganden können nach den im folgenden beschriebenen erfindungsgemäßen Verfahren aus folgenden kommerziell erhältlichen oder leicht zugänglichen Grundgerüsten dargestellt werden:

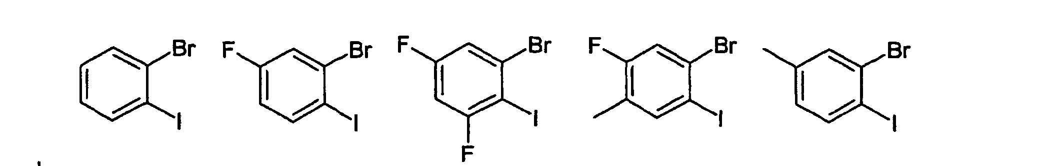

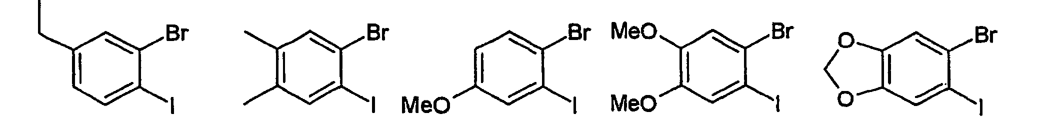

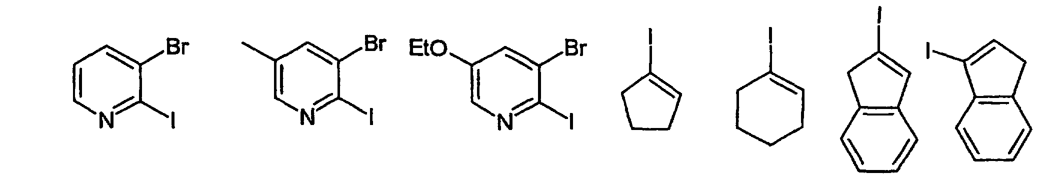

Für die Synthese von Verbindungen der Formeln (Ia) und (Ib) stehen unterschiedliche Verfahren zur Verfügung.

Die Auswahl eines entsprechenden Darstellungsverfahrens ist abhängig von der Verfügbarkeit der entsprechenden Edukte und vom gewünschten Substitutionsmuster. Nachfolgend sollen erfindungsgemäße Syntheseverfahren anhand von einfachen Beispielen beschrieben werden. Die Verfahren illustrieren die Vielfalt der mit diesen Verfahren erhältlichen Ligandsysteme. Besonders vorteilhaft an den erfindungsgemäßen Verfahren ist, daß viele Ligandsysteme einfach in wenigen Reaktionsschritten darstellbar sind.

### Darstellung der Bisphosphine

Die aliphatisch-aromatischen Grundgerüste werden bevorzugt durch eine Negishi-Kreuzkupplung von cyclischen Vinyliodiden und Halogenaromaten dargestellt. Die Einführung einer chiralen Phosphineinheit an das aliphatische System gelingt im Eintopfverfahren durch asymmetrische Hydroborierung mit einem chiralen Boran in Abwandlung der allgemeinen Literaturvorschrift (H. C. Brown et al. J. Org. Chem. 1982, *47*, 5074). Nachfolgende Transboronierung erwies sich zur Darstellung der erfindungsgemäßen Verbindungen als vorteilhaft. Nach einem erfindungsgemäßen Verfahren kann das chirale Boran mit Zinkdiorganylen ohne Racemisierung transmetalliert (Micouin, L.; Oestreich, M.; Knochel, P., Angew. Chem., Int. Ed. Engl. 1997, *36*, 245-246; A. Boudier, P. Knochel, Tetrahedron Lett. 1999, *40*, 687-690) und anschließend unter Retention der Konfiguration phosphiniert werden. Die Einführung der zweiten Phosphingruppe gelingt durch Brom/Lithiumaustausch mit starken Basen (z.B. Butyllithium) und anschließender Phosphinierung mit einem Chlorphosphan. Zur Reinigung der phosphorhaltigen Zwischenstufen und der Liganden werden die gängigen literaturbekannten Methoden verwendet. Eine Inversion am stereogenen Zentrum wird durch eine Variation des oben beschriebenen Verfahrens erreicht. Die asymmetrische Hydroborierung wird oxidativ aufgearbeitet und anschließend wird der chirale Alkohol mit einer basenstabilen Schutzgruppe blockiert. Ein Brom/Lithiumaustausch mit nachfolgender Phosphinierung und Entfernung der Schutzgruppe liefert einen chiralen Phosphinoalkohol. Eine nachfolgende Transformation in eine entsprechende Abgangsgruppe und Phosphonierung unter S_{N}2-Bedingungen ermöglicht die Bildung des cis-konfigurierten Bisphosphins unter kompletter Stereoinversion.

### Darstellung der Phosphin-Phoshinite und Phosphin-Phosphiten

Die erfindungsgemäßen Phosphin-Phosphinite und Phosphin-Phosphite lassen sich aus den chiralen Alkoholen herstellen. Die chiralen Alkohole sind über eine Negishi-Kupplung und nachfolgender asymmetrischen Hydroborierung mit oxidativer Aufarbeitung leicht zugänglich. Die nucleophile Ringöffnung eines meso-Epoxids mit nachfolgender Racematspaltung liefert ebenfalls einen schnellen Zugang zu chiralen Alkoholen, die als Grundbaustein der erfindungsgemäßen Liganden dienen. Die trans-konfigurierten Alkohole werden in Gegenwart einer Stickstoff-Base und eines Chlorphosphans zum Phosphinit umgesetzt. Diese Prozesse können durch die Verwendung von Grignardverbindungen als Base noch stark verbessert werden. Dies gilt insbesondere für die Umsetzung mit aliphatischen Chlorphosphanen. Die Einführung der zweiten Phosphingruppe gelingt durch Brom/Lithiumaustausch mit starken Lithiumbasen und anschließender Phosphinierung mit einem Chlorphosphan. Zur Darstellung der cis-konfigurierten Liganden muß eine Inversion des Stereozentrum durchgeführt werden. Die Inversion kann in einem Reaktionschritt mit Hilfe der Mitsunobu-Reaktion oder in einem zweistufigen Prozeß über eine Oxidation gefolgt von einer diastereoselektiven Reduktion erreicht werden. Nach der Inversion des Stereozentrums wird, nach dem oben beschrieben Weg, das Phosphinit gebildet und über einen Brom/Lithiumaustausch mit starken Lithiumbasen und anschließender Phosphinierung mit einem Chlorphosphan das cis-konfigurierte Phosphin-Phosphinit dargestellt.

In einem bevorzugten Verfahren zur Herstellung der Phosphin-Phosphinite auf 3,5-Dimethyl-thiophen Basis wird der chirale Alkohol wie oben beschrieben durch Epoxidöffnung und nachfolgender Racematspaltung erhalten und an der Hydroxyfunktion mit einer basenstabilen Schutzgruppe geschützt. Durch einen Brom/Lithiumaustausch, nachfolgender Phosphonierung und Entfernung der Schutzgruppe wird der chirale Phosphinoalkohol erhalten. In Gegenwart von Grignard-Reagenzien erfolgt die Bildung des Phosphin-Phosphinits analog zu dem oben beschriebenen Verfahren.

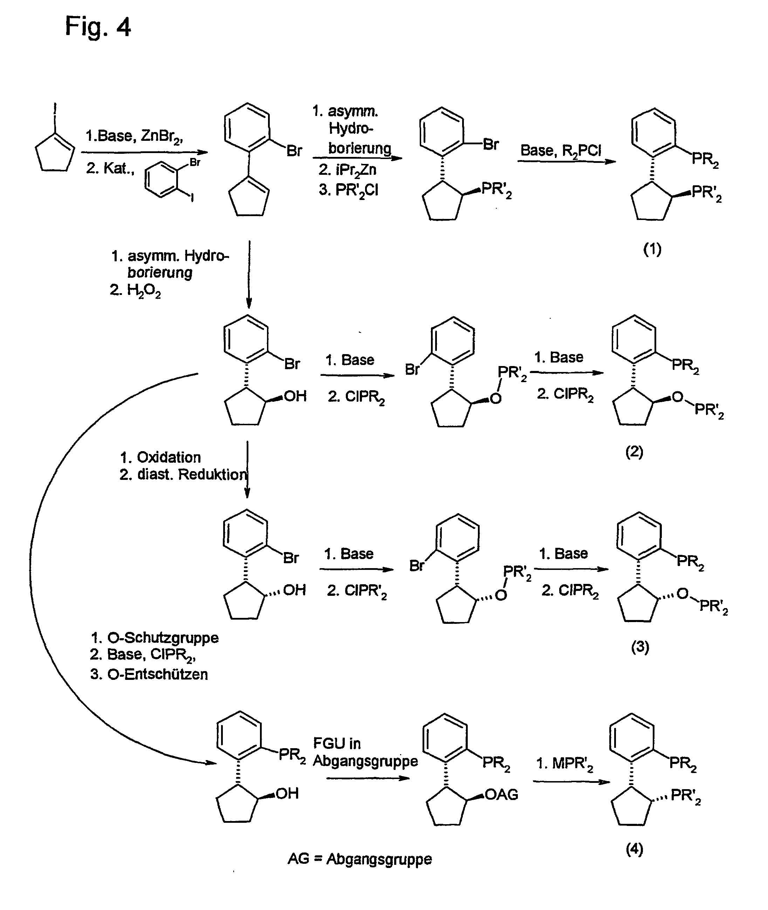

In Fig. 4 sind die einzelnen Syntheserouten in einer Übersicht dargestellt. In Fig. 4 bezeichnen die Substituentenplatzhalter R und R' allgemein verschiedene in der obigen Definition mit R1 - R2 genauer benannte Substituenten. Der Übersichtlichkeit halber wurden in den Illustrationen einfache Ligandgrundgerüste wie Phenylcyclopentyl und thiophenyl-tetrahydrofuranyl ausgewählt, ohne damit Einschränkungen oder Limitierungen zu implizieren.

Fig. 4 zeigt, daß die erfindungsgemäßen Liganden einfach synthetisch zugänglich sind und, dies obwohl die Verfahren eine Vielzahl unterschiedlicher Substituenten tolerieren müssen. Die Liganden sind in einem Dreischritt-Verfahren stereoselektiv herstellbar und dabei kann von einfachen Edukten ausgegangen werden. Dies eröffnet eine mögliche Synthese im technischen Maßstab.

Nachfolgend sollen die in Figur 4 skizzierten Darstellungswege für die erfindungsgemäßen Liganden anhand der bevorzugten erfindungsgemäßen Syntheseprozesse näher erläutert werden.

### Darstellung der Ligand-Grundgerüste:

### Syntheseweg A:

Die Synthese der aliphatisch-aromatischen Grundgerüste gelingt im Eintopfverfahren durch Negishi-Kreuzkupplung von literaturbekannten cyclischen Vinyliodiden und Halogenaromaten (Darstellung der Vinyliodide nach bekannten Methoden z.b. aus Ketonen mit Hydrazin/I₂/Base (Barton, D. K. Tetrahedron 1988, *44*, 147-62) oder LDA/ CIP(O)(OEt)₂/ISiMe₃ (Lee, K; Wiemer, D. F. Tetrahedron Lett. 1993, *34*, 2433-6).). (Schema 1).

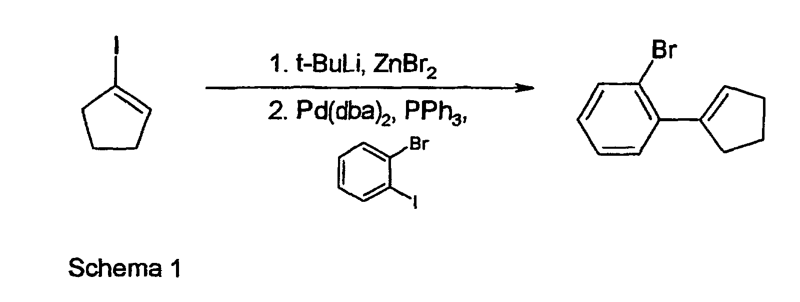

### Syntheseweg B:

Alternativ lassen sich die erfindungsgemäßen Grundgerüste auch durch basische Ringöffnung von meso-Epoxiden und nachfolgende Wassereliminierung darstellen. Eine Racematspaltung des racemischen Alkohol liefert den trans verknüpften enantiomerenreinen Alkohol (Schema 2).

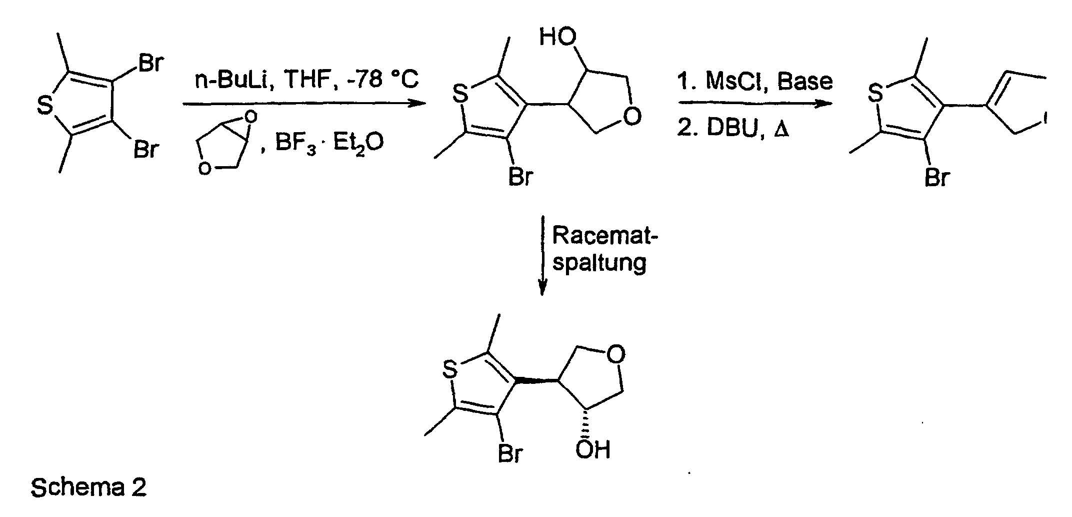

### Phosphinierungen

### Liganden mit trans-Konfiguration im Aliphaten

Die Einführung einer chiralen Phosphineinheit an das aliphatische System gelingt im Eintopfverfahren durch asymmetrische Hydroborierung mit einem chiralen Boran in Abwandlung der allgemeinen Literaturvorschrift (H. C. Brown et al. J. Org. Chem. 1982, *47*, 5074). Nachfolgende Transboronierung erwies sich zur Darstellung der erfindungsgemäßen Verbindungen als vorteilhaft. Nach einem erfindungsgemäßen Verfahren kann das chirale Boran mit Zinkdiorganylen ohne Racemisierung transmetalliert (Micouin, L.; Oestreich, M.; Knochel, P., Angew. Chem., Int. Ed. Engl. 1997, *36*, 245-246; A. Boudier, P. Knochel, Tetrahedron Lett. 1999, *40*, 687-690) und anschließend unter Retention der Konfiguration phosphiniert werden. Oxidative Aufarbeitung ermöglicht die Isolierung des gewünschten Produktes (Schema 3).

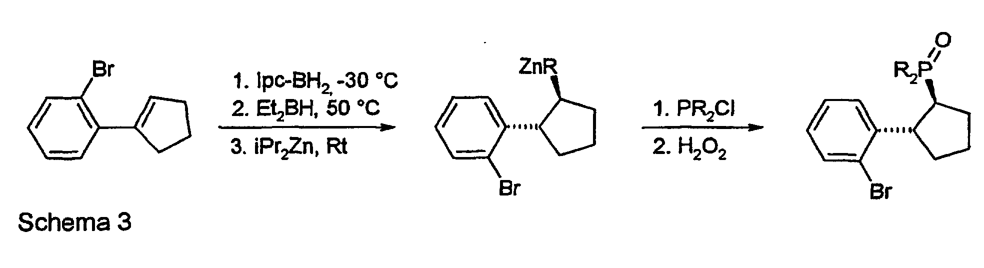

Die Einführung der Phosphineinheit an das aromatische System gelingt unter Variation literaturbekannter Methoden:
Als besonders vorteilhafte Prozessführung zur Synthese der erfindungsgemäßen Verbindungen hat sich die nachfolgend dargestellte Reaktionssequenz erwiesen. Vorteilhaft ist dabei die Durchführung aller Reaktionsschritte in einem Topf ohne zwischengeschaltete Aufarbeitung. (Schema 4). 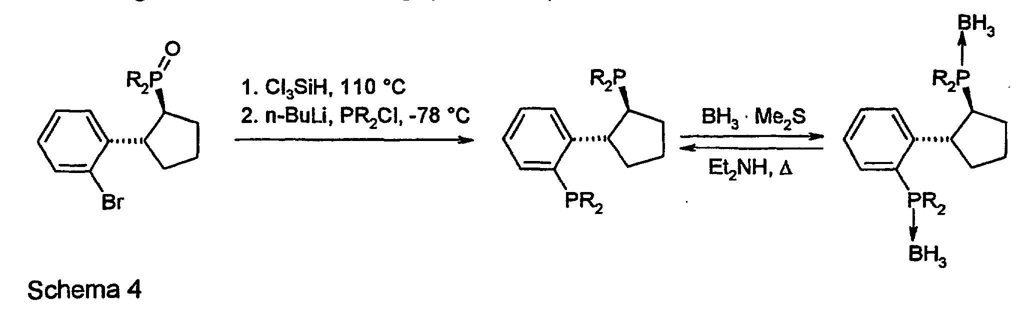 Das Phosphinoxid wird zunächst reduziert und der Aromat nach nachfolgendem Brom/Lithiumaustausch mit n-Butyllithium mit einem entsprechenden Chlorophosphan phosphiniert. Das entstandene Bisphosphan lässt sich nach erfindungsgemäßem Verfahren vorteilhaft als Boran-Addukt isolieren und nachfolgend wieder auf bekanntem Wege in das freie Phosphin umwandeln.

### Liganden mit cis-Konfiguration im Aliphaten

Alternativ wird das chirale Boran mit H₂O₂ in Abwandlung der allgemeinen Literaturvorschrift (H. C. Brown et al. J. Org. Chem. 1982, 47, 5074). in den entsprechenden trans-konfigurierten Alkohol umgewandelt, O-geschützt und am Aromaten phosphiniert. Freisetzung des Alkohols gelingt mit Fluorid. (Schema 5).

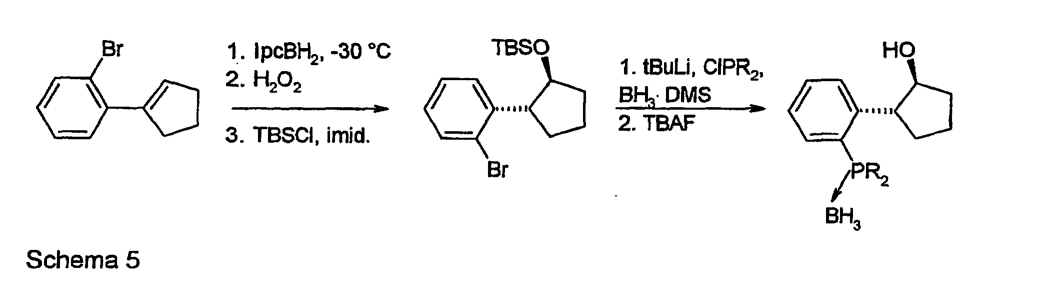

Nachfolgende Transformation in das entsprechende Mesylat ermöglicht nach prinzipiell bekanntem Reaktionsschema (z.B. U. Nagel, H.G. Nedden, Chem. Ber./Recueil, 1997, *130*, 385), die Umwandlung unter kompletter Stereoinversion in das chirale cis-konfigurierte Bisphosphin, welches nachfolgend durch eine Oxidations/Reduktionssequenz oder Boranschützung/Entschützung aufgereinigt werden kann (Schema 6).

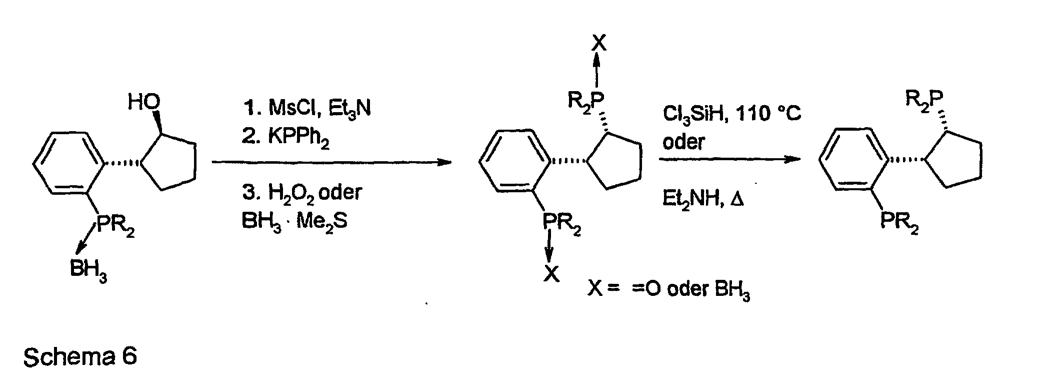

### Darstellung von Phosphin-Phosphiniten oder Phosphin-Phosphiten

Entsprechende erfindungsgemäße Phosphin-Phosphiniten und Phosphin-Phosphiten sind aus den chiralen Phosphinoalkoholen in einem Schritt durch Addition von Chlorphosphinen oder Chlorophosphiten in Gegenwart stöchiometrischer Mengen einer starken Base erhältlich. Dabei wurde überraschenderweise gefunden, daß die Addition von Chlorphosphan nach literaturbekannten Prozessen (Vorschriften für Substitutionsreaktionen dieses Typs: z. B. Reetz et al. Angew. Chem. 1999, *111*, 134; RajanBabu et al., J. Org. Chem. 1997, 62, 6012; Onuma et al. Bull. Chem. Soc. Jpn. 1980, 53, 2012) mit Triethylamin oder Pyridin als Base nicht oder nur sehr unzureichend erfolgt. Besonders aliphatische Chlorphosphane sind auf diesem Wege kaum darstellbar. Verwendung von EtMgBr als Base ermöglicht dagegen die Darstellung und Isolierung der erfindungsgemäßen Phosphin-Phosphinite in guten bis sehr guten Ausbeuten (Schema 7).

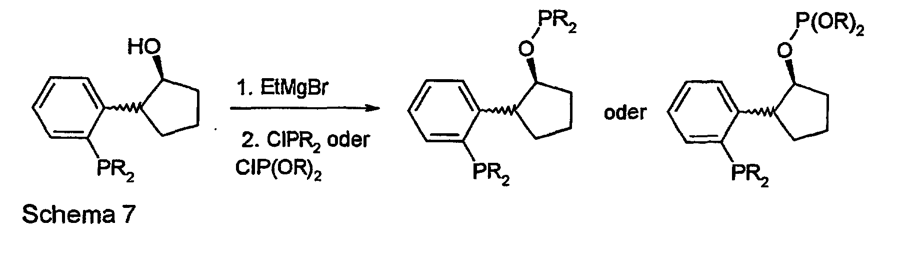

Erfindungsgemäß können Liganden, bei denen die Substituenten der beiden Phosphoreinheiten gleich sind, auf direkterem Wege durch gleichzeitige Einführung der Reste alternativ in einem Eintopfverfahren durch Umsetzung mit 2 Äquivalenten einer starken Base (z.B. n-Butyllithium oder tert.-Butyllithium) und nachfolgender Reaktion mit 2 Äquivalenten des entsprechenden Chlorphosphanes dargestellt werden (Schema 8).

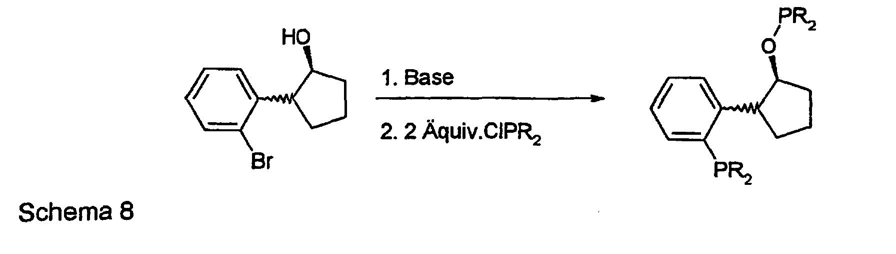

Die erfindungsgemäßen Phosphin-Phosphinite sind ebenso aus einer Kombination der oben beschriebenen Verfahren zugänglich (Schema 9).

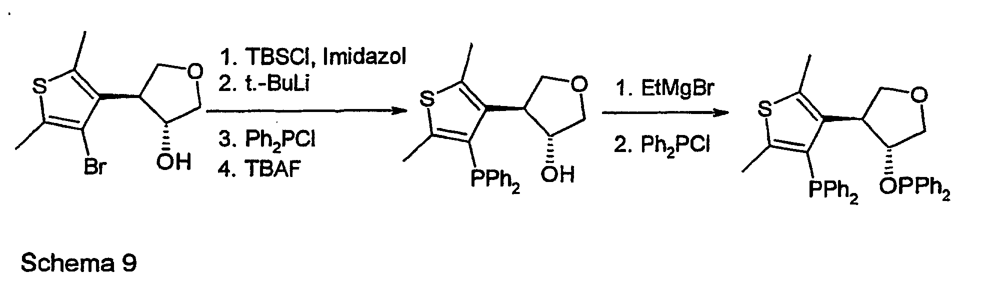

Die Verbindungen der allgemeinen Formeln (Ia) und (Ib) können als Liganden an Metallen in asymmetrischen, Metall-katalysierten Reaktionen (wie z.B. Hydrierung, der Hydroformylierung, der Umlagerung, der allylischen Alkylierung, der Cyclopropanierung, Hydrosilylierung, Hydridübertragungen, Hydroborierungen, Hydrocyanierungen, Hydrocarboxylierungen, Aldol Reaktionen oder Heck-Reaktion) sowie bei Polymerisationen eingesetzt werden. Sie sind insbesondere für asymmetrische Reaktionen gut geeignet.

Geeignete Komplexverbindungen, insbesondere der allgemeinen Formel (II), enthalten erfindungsgemäße Verbindungen der Formeln (Ia) und (Ib) als Liganden.

[MₓP_{y}L_{z}S_{q}]Aᵣ (II)

wobei in der allgemeinen Formel (II) M ein Metallzentrum, bevorzugt ein Übergangsmetallzentrum, L gleiche oder verschiedene koordinierende organische oder anorganische Liganden und P erfindungsgemäße bidentate Organophosphorliganden der Formeln (Ia) und (Ib) darstellen, S koordinierende Lösungsmittelmoleküle und A Äquivalente aus nicht koordinierenden Anionen repräsentiert, wobei x und y ganzen Zahlen größer oder gleich 1, z, q und r ganzen Zahlen größer oder gleich 0 sind.

Die Summe y + z + q wird durch die an den Metallzentren zur Verfügung stehenden Koordinationszentren nach oben begrenzt, wobei nicht alle Koordinationsstellen besetzt sein müssen. Bevorzugt sind Komplexverbindungen mit oktaedrischer, pseudo-oktaedrischer, tetraedrischer, pseudo-tetraedrischer, quadratisch-planarer Koordinationssphäre, die auch verzerrt sein kann, um das jeweilige Übergangsmetallzentrum. Die Summe y + z + q ist in solchen Komplexverbindungen kleiner oder gleich 6x.

Die erfindungsgemäßen Komplexverbindungen enthalten mindestens ein Metallatom oder -ion, vorzugsweise ein Übergangsmetallatom oder -ion, insbesondere aus Palladium, Platin, Rhodium, Ruthenium, Osmium, Iridium, Kobalt, Nickel, oder/und Kupfer.

Bevorzugt sind Komplexverbindungen mit weniger als vier Metallzentren, besonders bevorzugt solche mit ein oder zwei Metallzentren. Die Metallzentren können dabei mit verschiedenen Metallatomen und/oder -ionen besetzt sein.

Bevorzugte Liganden L solcher Komplexverbindungen sind Halogenid, besonders Cl, Br und I, Dien, besonders Cyclooctadien, Norbomadien, Olefin, besonders Ethylen und Cycloocten, Acetato, Trifluoracetato, Acetylacetonato, Allyl, Methallyl, Alkyl, besonders Methyl und Ethyl, Nitril, besonders Acetonitril und Benzonitril, sowie Carbonyl und Hydrido Liganden.

Bevorzugte koordinierende Lösungsmittel S sind Amine, besonders Triethylamin, Alkohole, besonders Methanol und Aromaten, besonders Benzol und Cumol.

Bevorzugte nichtkoordinierende Anionen A sind Trifluoracetat, Trifluormethansulfonat, BF₄; ClO₄, PF₆, SbF₆, und BAr₄.

In den einzelnen Komplexverbindungen können dabei unterschiedliche Moleküle, Atome oder Ionen der einzelnen Bestandteile M, P, L, S und A enthalten sein.

Bevorzugt unter den ionisch aufgebauten Komplexverbindungen sind Verbindungen des Typs [RhP(Dien)]⁺A⁻, wobei P einen erfindungsgemäßen Liganden der Formeln (Ia) und (Ib) repräsentiert.

Die Herstellung dieser Metall-Ligand-Komplexverbindungen kann in situ durch Reaktion eines Metallsalzes oder eines entsprechenden Vorkomplexes mit den Liganden der allgemeinen Formeln (Ia) und (Ib) erfolgen. Darüber hinaus kann eine Metall-Ligand-Kompiexverbindung durch Reaktion eines Metallsalzes oder eines entsprechenden Vorkomplexes mit den Liganden der allgemeinen Formeln (Ia) und (Ib) und anschließende Isolierung gewonnen werden.

Beispiele für die Metallsalze sind Metallchloride, -bromide, -iodide, -cyanide, -nitrate, - acetate, -acetylacetonate, -hexafluoracetylacetonate, tetrafluoroborate, - perfluoracetate oder -triflate, insbesondere des Palladium, Platins, Rhodium, Ruthenium, Osmium, Iridium, Kobalts, Nickels oder/und des Kupfers.
Beispiele für die Vorkomplexe sind:
Cyclooctadienpalladiumchlorid, Cyclooctadienpalladiumiodid,
1,5-Hexadienpalladiumchlorid, 9,5-Hexadienpalladiumiodid,
Bis(dibenzylidenaceton)palladium, Bis(acetonitril)palladium(II)chlorid, ,
Bis(acetonitril)palladium(II)bromid, Bis(benzonitril)palladium(II)chlorid,
Bis(benzonitril)palladium(II)bromid, Bis(benzonitril)palladium(II)iodid,
Bis(allyl)palladium, Bis(methallyl)palladium, Allylpalladiumchlorid-Dimer,
Methallylpalladiumchlorid-Dimer, Tetramethylethylendiaminpalladiumdichlorid,
Tetramethylethylendiaminpalladiumdibromid,
Tetramethylethylendiaminpalladiumdiiodid,
Tetramethylethylendiaminpalladiumdimethyl,
Cyclooctadienplatinchlorid, Cyclooctadienplatiniodid, 1,5-Hexadienplatinchlorid,
1,5-Hexadienplatiniodid, Bis(cyclooctadien)platin, Kalium(ethylentrichloroplatinat),
Cyclooctadienrhodium(I)chlorid-Dimer, Norbornadienrhodium(I)chlorid-Dimer,
1,5-Hexadienrhodium(I)chlorid-Dimer, Tris(triphenylphosphan)rhodium(I)chlorid,
Hydridocarbonyltris(triphenylphosphan)rhodium(I)chlorid,
Bis(cyclooctadien)rhodium(I)perchlorat, Bis(cyclooctadien)rhodium(I)tetrafluorborat,
Bis(cyclooctadien)rhodium(I)triflat, Bis(acetonitrilcyclooctadien)rhodium(I)perchlorat,
Bis(acetonitrilcyclooctadien)rhodium(I)tetrafluorborat,
Bis(acetonitrilcyclooctadien)rhodium(I)triflat,
Cyclopentadienrhodium(III)chlorid-Dimer,
Pentamethylcyclopentadienrhodium(III)chlorid-Dimer,
(cyclooctadien)Ru(η³-allyl)₂, ((cyclooctadien)Ru)₂(acetat)₄,
((Cyclooctadien)Ru)₂(trifluoracetat)₄, RuCl₂(Aren)-Dimer,
Tris(triphenylphosphan)ruthenium(II)chlorid, Cyclooctadienruthenium(II)chlorid,
OsCl₂(Aren)-Dimer, Cyclooctadieniridium(I)chlorid-Dimer,
Bis(cycloocten)iridium(I)chlorid-Dimer,
Bis(cyclooctadien)nickel, (Cyclododecatrien)nickel, Tris(norbornen)nickel,
Nickeltetracarbonyl, Nickel(II)acetylacetonat,
(Aren)kupfertriflat, (Aren)kupferperchlorat, (Aren)kupfertrifluoracetat, Kobaltcarbonyl.

Die Komplexverbindungen auf Basis von ein oder mehreren Metallen der metallischen Elemente, insbesondere aus der Gruppe von Ru, Co, Rh, Ir, Ni, Pd, Pt, Cu, können bereits Katalysatoren sein oder zur Herstellung von Katalysatoren auf Basis eines oder mehrerer Metalle der metallischen Elemente, insbesondere aus der Gruppe von Ru, Co, Rh, Ir, Ni, Pd, Pt, Cu verwendet werden. Alle diese Komplexverbindungen sind besonders geeignet in der asymmetrischen Hydrierung von C=C-, C=O- oder C=N-Bindungen, in denen sie hohe Aktivitäten und Selektivitäten aufweisen und in der asymmetrischen Hydroformylierung. Insbesondere erweist es sich hier als vorteilhaft, daß sich die Liganden der allgemeinen Formeln (Ia) und (Ib) durch ihre einfache, breite Abwandelbarkeit sterisch und elektronisch sehr gut auf das jeweilige Substrat und die katalytische Reaktion abstimmen lassen.

Entsprechende Katalysatoren enthalten mindestens eine der erfindungsgemäßen Komplexverbindungen.

Im folgenden werden einige nicht einschränkende Beispiele zur Verdeutlichung der Erfindung gegeben.

### Beispiele

### Allgemeines

Reaktionen luftempfindlicher Verbindungen wurden in einer argongefüllten Glove-Box oder in Standard Schlenkrohren durchgeführt. Lösungsmittel Tetrahydrofuran (THF), Diethylether und Dichlormethan wurden entgast und mittels einer Lösungsmitteltrocknungsanlage (Innovative Technologies) durch Filtration durch eine mit aktiviertem Aluminiumoxid gefüllte Säule absolutiert, Toluol und Pentan wurden zusätzlich durch eine mit einem Kupferkatalysator gefüllte Säule von Sauerstoff befreit.

Die folgenden Beispiele dienen zur Erläuterung der Erfindung. Sie sollen in keiner Weise eine Beschränkung darstellen.

### Beispiel 1 1-(Cyclopenten-1-yl)-2-brombenzol

Zu Cyclopentenyliodid (16 mmol) in THF (16 ml) wird bei -78 °C langsam t-BuLi (32 mmol, 2 Äquiv) gegeben. Nach 1 h bei dieser Temperatur wird eine 1 M Lösung von ZnBr₂ in THF (16 ml) dazugegeben. Es wird 30 min bei -40 °C und weitere 30 min bei RT gerührt.
Parallel werden Pd(dba)₂ (6 mol %) und PPh₃ (12 Mol%) in THF (16 ml) 10 min bei RT gerührt. Zu dieser Lösung wird das 2-Brom-1-lodbenzol (16 mmol, 1 Äquiv.) gegeben. Es wird erneut 10 min bei RT gerührt. Die so erhaltene Lösung wird zu der anderen Lösung überkannuliert. Es wird 12 h bei 50 °C gerührt. Nach extraktiver Aufarbeitung (Et₂O/NaCl) wird das gewünschte Produkt 1-(cyclopenten-1-yl)-2-brombenzol aus der etherischen Phase erhalten, säulenchromatographisch (Pentan) gereinigt und in 61 % Ausbeute isoliert.
¹H-NMR (CDCl₃):
δ = 7.43 (d, 1 H), 7.20 - 7.10 (m, 2H), 6.87 (m, 1 H), 5.88 (d, 1 H), 2.65 (m, 2H), 2.43 (m, 2H), 1.95 (m, 2H) ppm.

### Beispiel 2 1-(Cyclopentyl-2-diphenylphosphinoxy)-2-brom)benzol

1-(Cyclopenten-1-yl)-2-brombenzol (1 mmol) in THF (0.5 ml) wird über einen Zeitraum von 30 min zu einer Lösung von frisch hergestelltem IpcBH₂ (1M in THF, 1 ml) bei -30 °C gegeben. Es wird 2 Tage bei dieser Temperatur gerührt. Das Lösungsmittel wird nun im Hochvakuum entfernt und das erhaltene Boran mit Et₂BH-Lösung (1 ml, 7.3 M in DMS) 12 h bei 50 °C umgesetzt. Nach Entfernung des Lösungsmittels im Hochvakuum wird Zn(iPr)₂ (2.25 ml, 4 M in Et₂O) dazugegeben. Die erhaltene Lösung wird 5 h bei RT gerührt. Nach 3 h im Hochvakuum wird der gräulich-schwarze Rückstand des Kolbens in THF (2 ml) aufgenommen und unter Argon zentrifugiert. Die überstehende klare Lösung wird überkannuliert, auf 0 °C gekühlt und langsam mit Ph₂PCl (4 mmol) versetzt. Es wird 4 Tage bei RT gerührt. Nach vorsichtiger Zugabe von H₂O₂ (1ml, 30 %ig) wird 30 min bei RT gerührt. Extraktive Aufarbeitung (CH₂Cl₂/NaCl) und säulenchromatographische Reinigung (CH₂Cl₂/MeOH 49:1) ergaben das gewünschte diastereomerenreine Produkt in 45 % Gesamtausbeute.
¹H-NMR (CD₃OD):
δ = 7.92 - 7.30 (m, 14H), 4.05 (m, 1 H), 3.41 (m, 1 H), 2.33 - 1.51 (m, 6H) ppm.
³¹P-NMR (CD₃OD):
δ = 38.7 (s, 1P)ppm.

### Beispiel 3 1-(Cyclopentyl-[2-diphenylphosphinoxy])-2-diphenylphosphinobenzol

1-(Cyclopentyl-2-diphenylphosphinoxy)-2-brom)benzol (0.4 mmol) wird in 9 ml Toluol gelöst und mit Cl₃SiH (10 Äquiv.) für 12 h refluxiert. Nach dieser Zeit wird für 2 h Hochvakuum angelegt. Es wird erneut Toluol (10 ml) und entgaste KOH-Lösung (2M, 10 ml) dazugegeben. Nach Entfernen der wässrigen Phase wird über MgSO₄ getrocknet. Die Suspension wird unter Argon filtriert, das Lösungsmittel im Hochvakuum entfernt und der farblose Rückstand in THF (6 ml) aufgenommen. Die Lösung wird auf -78 °C gekühlt und mit n-BuLi (1.2 Äquiv.) versetzt. Nach 2 h bei dieser Temperatur wird Ph₂PCl (1.2 Äquiv.) zugegeben. Langsames Auftauen auf RT (7 h) wird gefolgt von der Zugabe von BH₃ ^{·}DMS (10 Äquiv.) Die Lösung wird 12 h bei RT gerührt. Nach Aufarbeitung in CH₂Cl₂/NaCl-Lösung wird das Rohprodukt säulenchromatographisch (CH₂Cl₂/Pentan, dann CH₂Cl₂) gereinigt und in einer Gesamtausbeute von 76 % erhalten.
¹H-NMR (CDCl₃) Boran-Addukt:
δ = 7.62 - 6.90 (m, 24H), 4.25 (m, 1H), 3.31 (m, 1 H), 2.18 - 1.01 (m, 6H) ppm.
³¹P-NMR (CDCl₃) Boran-Addukt:
δ = 25.0(s, 1 P), 20.5 (s, 1 P) ppm.
³¹P-NMR (CDCl₃):
δ = - 4.2 (d, *J* = 5.7 Hz, 1 P), -17.4 (d, *J* = 5.7 Hz, 1 P) ppm.

### Beispiel 4 trans-4-(-4-Brom-2,5-dimethyl-3-thienyl)tetrahydro-3-furanol

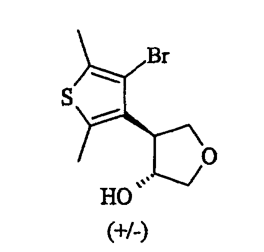

Zu einer auf -78° abgekühlten Lösung von 3,4-Dibrom-2,5-dimethylthiophen (0.239 mol) in 325 ml THF wird tropfenweise n-Butyllithium (1.6M in Hexan; 0.239 mol) zugegeben und 30 min gerührt. Zu dieser Lösung werden tropfenweise 3,4-Epoxytetrahydrofuran (0.217 mol) und Bortrifluorid-Etherat (0.217 mol) zugegeben. Die Reaktionslösung wird auf 0°C erwärmt und weitere 3h gerührt. Anschließend wird mit ges. Ammoniumchlorid-Lösung hydrolysiert und die wäßrige Phase zweimal mit tert.-Butylmethylether extrahiert. Nach dem Trocknen wird das Lösungsmittel im Vakuum entfernt und das Rohprodukt mittels Chromatographie gereinigt. Das Produkt wird als Öl in einer Ausbeute von 64% erhalten.
¹H NMR (CDCl₃):
δ = 4.82 (q, 1 H), 4.29 - 4.21 (m, 2H), 4.11 (t, 1 H), 3.99 (dd, 1 H), 3.67 -3.60 (m, 1 H), 2.50 (s, 3H), 2.41 (s, 3H) ppm.

### Beispiel 5 (3R, 4S)-4-(Brom-2,5-dimethyl-3-thienyl)-tetrahydro-3-furanol und (3S, 4R)-4-(Brom-2,5-dimethyl-3-thienyl)-tetrahydro-3-furanol-acetat

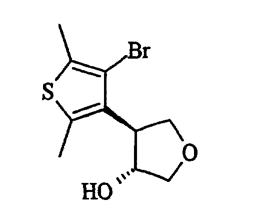

Zu einer Lösung von trans-4-(-4-Brom-2,5-dimethyl-3-thienyl)tetrahydro-3-furanol (0.153 mol) in 425 ml Vinylacetat wird festes Novozym 435 gegeben und anschließend 24 h gerührt. Das Enzym wird durch Filtration entfernt und der Rückstand mit tert.-Butylmethylether gewaschen. Nach dem Entfernen des Lösungsmittels werden die beiden Produkte mittels Chromatographie getrennt. (3R, 4S)-4-(Brom-2,5-dimethyl-3-thienyl)- tetrahydro-3-furanol wird in einer Ausbeute von 49% (>99%ee) und (3S, 4R)-4-(Brom-2,5-dimethyl-3-thienyl)- tetrahydro-3-furanolacetat in einer Ausbeute von 50% (>97%ee) erhalten.
¹H NMR (CDCl₃):
δ = 5.65 - 5.60 (m, 1 H), 4.44 (dd, 1 H), 4.26 (t, 1 H), 4.07 (d, 1 H), 4.03 (d, 1 H), 3.82 (ddd, 1 H), 2.51 (s, 3H), 2.46 (s, 3H), 2.17 (s, 3H) ppm.

### Beispiel 6 (3R, 4S)-4-(Brom-2,5-dimethyl-3-thienyl)-tetrahydro-3-furanyl-tert-butyldimethylsilylether

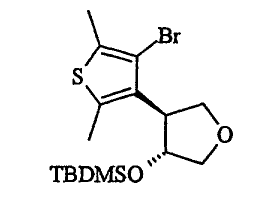

Zu einer auf 0°C gekühlten Lösung von (75 mmol) in 200 ml Dichlormethan werden tert.-Butyldimethylsilylchlorid (82 mmol) und Imidazol (97 mmol) hinzugegeben und die Reaktionslösung über Nacht auf Raumtemperatur erwärmt Die Reaktionslösung wird mit 150 ml 1 M Salzsäure hydrolysiert und die wäßrige Phase dreimal mit Dichlormethan extrahiert. Die vereinigten organischen Phasen werden mit ges. Natriumhydrogencarbonat-Lösung gewaschen. Nach dem Trocknen wird das Lösungsmittel im Vakuum entfernt und das Produkt als oranges Öl in quantitativer Ausbeute erhalten.
¹H NMR (CDCl₃):
δ = 4.9 (q, 1 H), 4.28 - 4.18 (m, 3H), 3.82 (dd, 1 H), 3.68 (ddd, 1 H), 2.54 (s, 3H), 2.47 (s, 3H), 0.96 (s, 9H), 0.04 (s, 3H), 0.00 (s, 3H) ppm.

### Beispiel 7 1-(t-Butyl-1,1-dimethylsilyl-((3R,4S)4-(4-(1,1-di(3,5-di(trifluormethyl)phenyl)phosphino)-2,5-dimethyl-3-thienyl)tetrahydro-3-furanyl)ether

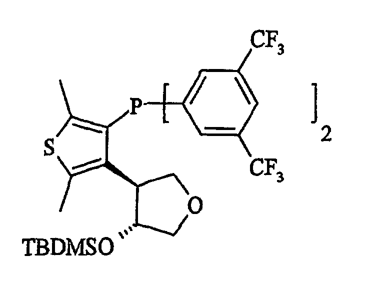

Zu einer Lösung von (3R, 4S)-4-(Brom-2,5-dimethyl-3-thienyl)-tetrahydro-3-furanyltert-butyl-dimethylsilylether (12.8 mmol) in 50 ml THF wird bei -78°C tert.-Butyllithium (25.5 mmol) über 10 min zugegeben und anschließend 1 h gerührt. Zu dieser Lösung werden 15.3 mmol Bis-(3,5-di-(trifluormethyl)-phenyl)chlorphosphan tropfenweise zugegeben und die Reaktionslösung über Nacht auf R.T. erwärmt. Anschließend wird mit entgastem Wasser hydrolysiert, die Lösung mit Dichlormethan verdünnt und die wäßrige Phase zweimal mit Dichlormethan extrahiert. Nach dem Trocknen und Entfernen des Lösungsmittels wird das Rohprodukt mittels Chromatographie gereinigt und aus kaltem Pentan kristallisiert. Das Produkt wird in 22% Ausbeute erhalten.
¹H NMR (C₆H₆):
δ = 7.86 (t, 4H), 7.79 (s, 2H), 4.92-4.99 (m, 1 H), 4.41 (dd, 1 H), 4.30 - 4.23 (m, 1 H), 4.11 - 4.01 (m, 2H), 3.97 (dd, 1 H,), 2.22 (s, 3H), 1.60 (s, 3H), 0.99 (s, 9H), 0.00 (s, 3H), -0.04 (s, 3H) ppm.

### Beispiel 8 1-(t-Butyl-1,1-dimethylsilyl-((3R,4S)4-(4-(1,1-di(o-toluol)phosphino)-2,5-dimethyl-3-thienyl)tetrahydro-3-furanyl)ether

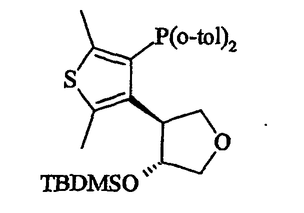

Die Synthese erfolgt analog zu Beispiel 7
Ausbeute: 59%
¹H NMR (C₆H₆):
δ = 7.00-7.30 (m, 8H), 4.90-5.11 (m, 1 H), 4.52 (dd, J = 10, 8 Hz, 1 H), 4.15-4.40 (m, 3H), 4.07 (dd, J = 10, 3 Hz, 1 H), 2.51 (s, 3H), 2.47 (s, 3H), 2.40 (s, 3H), 1.88 (s, 3H), 1.06 (s, 9H), 0.00 (s, 6H) ppm.
³¹P NMR (C₆H₆):
δ = -22.0 ppm.

### Beispiel 9 1-(t-Butyl-1,1-dimethylsilyl-((3R,4S)4-(4-(1,1-di(p-(fluoro))phenyl)phosphino)-2,5-dimethyl-3-thienyl)tetrahydro-3-furanyl)ether

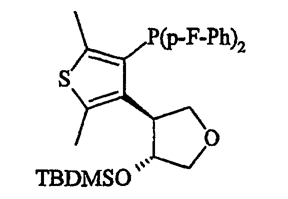

Die Synthese erfolgt analog zu Beispiel 7
Ausbeute: 37%
¹H NMR (C₆H₆):
δ = 7.43-7.52 (m, 4H), 7.27 (q, J = 8 Hz, 4H), 4.98-5.03 (m, 1 H), 4.48 (dd, J = 10, 8 Hz, 1H), 4.32 (dt, J = 9, 2 Hz, 1 H), 4.17 (t, J = 9 Hz, 1 H), 4.00-4.08 (m, 1 H), 3.93 (dd, J = 8, 3 Hz, 1 H), 2.69 (s, 3H), 2.01 (s, 3H), 1.01 (s, 9H), 0.03 (s, 3H), 0.00 (s, 3H) ppm.
³¹P NMR (C₆H₆):
δ = -25.3 ppm.

### Beispiel 10 1-(t-Butyl-1,1-dimethylsilyl-((3R,4S)4-(4-(1,1-di(3,5-di(methyl)phenyl)phosphino)-2,5-dimethyl-3-thienyl)tetrahydro-3-furanyl)ether

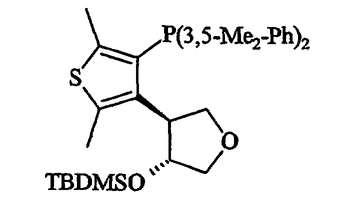

Die Synthese erfolgt analog zu Beispiel 7
Ausbeute: 6%
¹H NMR (C₆H₆):
δ = 7.34 (t, J = 8 Hz, 4H), 6.88 (s, 1 H), 6.84 (s, 1 H), 5.23-5.28 (m, 1 H), 4.64 (dd, J = 10, 8 Hz, 1 H), 4.57 (t, J = 9 Hz, 1 H), 4.40 (dt, J = 8, 2 Hz, 1 H), 4.07-4.19 (m, 2H), 2.34 (s, 3H), 2.20 (s, 6H), 2.14 (s, 6H), 2.12 (s, 3H), 1.02 (s, 9H), 0.03 (s, 3H), 0.00 (s, 3H) ppm.
³¹P NMR (C₆H₆):
δ = -10.9 ppm.

### Beispiel 11

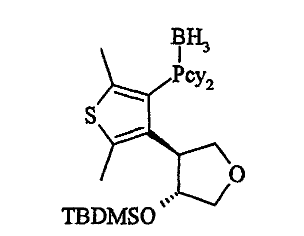

Zu einer Lösung von (3R, 4S)-4-(Brom-2,5-dimethyl-3-thienyl)- tetrahydro-3-furanyltert-butyl-dimethylsilylether (12.8 mmol) in 50 ml THF wird bei -78°C tert.-Butyllithium (25.5 mmol) über 10 min zugegeben und anschließend 1 h gerührt. Zu dieser Lösung werden 15.3 mmol Dicyclohexylchlorphosphan tropfenweise zugegeben und die Reaktionslösung über 2h auf R.T. erwärmt. Anschließend wird die Lösung erneut auf - 78°C abgekühlt und 5 Äquivalenten Boran (1 M in THF) zugegeben. Anschließend wird die Lösung auf R.T. erwärmt und mit entgastem Wasser hydrolysiert, die Lösung mit Dichlormethan verdünnt und die wäßrige Phase zweimal mit Dichlormethan extrahiert. Nach dem Trocknen und Entfernen des Lösungsmittels, wird das Rohprodukt mittels Chromatographie gereinigt und aus kaltem Pentan kristallisiert. Das Produkt wird in 58% Ausbeute erhalten.
¹H NMR (C₆H₆):
δ = 4.89 (q, J = 7 Hz, 1 H), 4.17-4.28 (m, 1 H), 4.63 (t, J = 8 Hz, 1 H), 4.20-4.26 (m, 1 H), 3.93 (t, J = 8 Hz, 1 H), 3.76 (dd, J = 9, 8 Hz, 1 H), 2.44 (s, 3H), 2.29 (s, 3H), 2.13-2.39 (m, 2H), 1.09-2.00 (m, 20H), 0.97 (s, 9H), 0.00 (s, 6H) ppm.
³¹P NMR (C₆H₆):
δ = 39.1 ppm.

### Beispiel 12 (3R, 4S)-4-(4-{1,1-di[3,5-di(trifluormethyl)phenyl]phosphino}-2,5-dimethyl-3-thienyl)tetrahydro-3-furanol

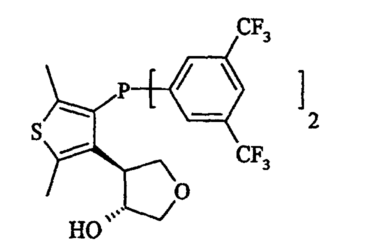

Tetrabutylammoniumfluorid-Lösung (5.7 mmol) wird tropfenweise bei 0°C zu einer Lösung von 1-(t-Butyl-1,1-dimethylsilyl-((3R,4S)4-(4-(1,1-di(3,5-di(trifluormethyl)phenyl)phosphino)-2,5-dimethyl-3-thienyl)tetrahydro-3-furanyl)ether (2.86 mmol) in 22 ml Tetrahydrofuran gegeben und über Nacht auf R.T. erwärmt. Das Lösungsmittel wird im Vakuum entfernt und das Rohprodukt mittels Chromatogrphie gereinigt und mit 87% Ausbeute isoliert.
¹H NMR (C₆H₆):
δ = 8.08 (t, 6H), 7.97 (s, 1 H), 7.92 (s, 1 H), 4.45 - 4.38 (m, 1 H), 4.10 (dd, 1 H), 4.01 - 3.92 (m, 1 H), 3.89 - 3.82 (m, 1 H), 3.58 (dd, 1 H), 2.29 (s, 3H), 2.15 (s, 3H) ppm.
³¹P NMR (C₆H₆)
δ = -9.93 (s, 1P) ppm.

### Beispiel 13 (3R, 4S)-4-(4-{1,1-di[o-toluol]phosphino}-2,5-dimethyl-3-thienyl)tetrahydro-3-furanol

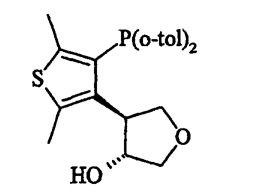

Die Synthese erfolgt analog zu Beispiel 12
Ausbeute: 93%
¹H NMR (CDCl₃):
δ = 7.46-7.54 (m, 4H), 7.38 (t, J = 7 Hz, 2H), 7.29 (dd, J = 8, 6 Hz, 1H), 7.20 (dd, J = 8, 6 Hz, 1 H), 4.68-4.84 (m, 1 H), 4.34-4.41 (m, 1 H), 4.03-4.12 (m, 2H), 3.83-3.93 (m, 2H), 2.69 (s, 3H), 2.57 (s, 3H), 2.56 (s, 3H), 2.26 (s, 3H), 1.60 (br s, 1 H) ppm.

### Beispiel 14 (3R, 4S)-4-(4-{1,1-di[3,5-di(methyl)phenyl]phosphino}-2,5-dimethyl-3-thienyl)tetrahydro-3-furanol

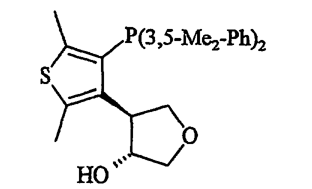

Die Synthese erfolgt analog zu Beispiel 12
Ausbeute: 99%
¹H NMR (CDCl₃):
δ = 7.32 (d, J = 7 Hz, 2H), 7.30 (d, J = 7 Hz, 2H), 6.78 (s, 2H), 4.64 (q, J = 7 Hz, 1H), 4.22 (dd, J = 11, 9 Hz, 1 H), 4.00-4.09 (m, 2H), 3.88-3.96 (m, 1H), 3.76 (dd, J = 11, 4 Hz, 1 H), 2.40 (s, 3H), 2.27 (s, 3H), 2.10 (s, 6H), 2.08 (s, 6H) ppm.

### Beispiel 15 (3R, 4S)-4-(4-{1,1-di[3,5-di(p-(fluoro)phenyl)]phosphino}-2,5-dimethyl-3-thienyl)tetrahydro-3-furanol

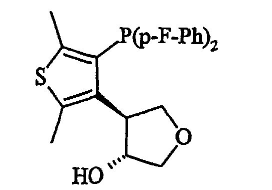

Die Synthese erfolgt analog zu Beispiel 12
Ausbeute: 99%
¹H NMR (C₆H₆):
δ = 7.30-7.40 (m, 4H), 6.93-7.02 (m, 4H), 4.72-4.77 (m, 1 H), 4.33 (dd, J = 11, 9 Hz, 1 H), 4.09-4.20 (m, 2H), 3.98-4.06 (m, 1 H), 3.89 (dd, J = 10, 4 Hz, 1 H), 2.43 (s, 3H), 2.10 (s, 3H), 1.32 (br s, 1 H) ppm.

### Beispiel 16

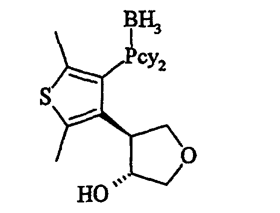

Die Synthese erfolgt analog zu Beispiel 12
Ausbeute: 76%
¹H NMR (C₆H₆):
δ = 4.28 (t, J = 9 Hz, 1 H), 4.18-4.24 (m, 1 H), 3.90-4.03 (m, 1 H), 3.87 (dd, J = 11, 8 Hz, 1H), 3.70 (t, J = 7 Hz, 1H), 3.56 (dd, J = 10, 5 Hz, 1 H), 2.70 (s, 3H), 2.50-2.64 (m, 1 H), 2.12-2.23 (m, 1 H), 2.03 (s, 3H), 0.88-1.98 (m, 20H) ppm.

### Beispiel 17 trans-4-(-4-Brom-2,5-dimethyl-3-thienyl)tetrahydro-3-pyranol

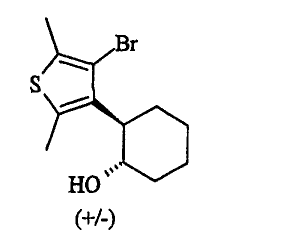

Die Synthese erfolgt analog zu Beispiel 4
Ausbeute: 64%
¹H NMR (CDCl₃):
δ = 4.25-4.62 (br m, 1 H), 2.92-3.11 (br m, 1H), 2.64 (br s, 3H), 2.55 (s, 3H), 2.30-2.40 (m, 1 H), 1.48-2.11 (m, 7H) ppm.

### Beispiel 18 (3R, 45)- und (3S, 4R)-4-(Brom-2,5-dimethyl-3-thienyl)-tetrahydro-3-pyranol-acetat

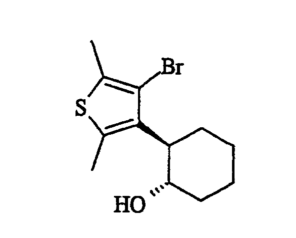

Die Synthese erfolgt analog zu Beispiel 5 unter Verwendung von ChiroCLEC PC
¹H NMR (CDCl₃):
δ = 5.40-5.54 (br m, 1H), 3.03-3.15 (br m, 1 H), 2.50 (s, 3H), 2.42, (s, 3H), 2.20-2.28 (m, 1 H), 1.91 (s, 3H), 1.81-2.00 (m, 3H), 1.38-1.66 (m, 4H) ppm.

### Beispiel 19 (3R, 4S)-4-(Brom-2,5-dimethyl-3-thienyl)- tetrahydro-3-pyranyl-tertbutyl-dimethylsilylether

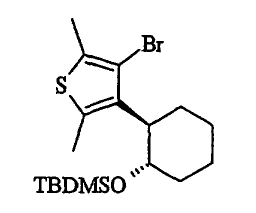

Die Synthese erfolgt analog zu Beispiel 6
Ausbeute: 78%
¹H NMR (CDCl₃):
δ = 4.13-4.28 (m, 1H), 2.73-2.90 (m, 1 H), 2.43 (s, 3H), 2.38 (s, 3H), 2.03-2.10 ( m, 1 H), 1.66-1.88 (m, 3H), 1.27-1.48 (m, 4H), 0.78 (s, 9H), 0.00 (s, 3H), -0.28 (s, 3H) ppm.

### Beispiel 20

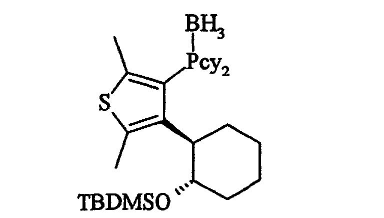

Die Synthese erfolgt analog zu Beispiel 11
Ausbeute: 78%
¹H NMR (CDCl₃):
δ = 3.93 (dt, J = 10, 4 Hz, 1 H), 2.40 (s, 3H),2.37-2.41 (m, 1 H), 2.16 (s, 3H), 2.01-2.12 (m, 1H), 0.90-1.96 (m, 29H), 0.80 (s, 9H), 0.00 (s, 3H), -0.18 (s, 3H) ppm.

### Beispiel 21

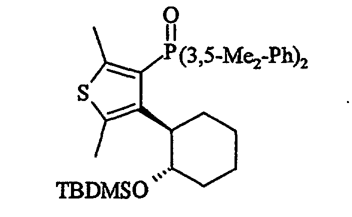

Zu einer Lösung von (3R, 4S)-4-(Brom-2,5-dimethyl-3-thienyl)-2-cyclohexyl-tert-butyldimethylsilylether (5.0 mmol) in 20 ml THF wird bei -78°C tert.-Butyllithium (10.0 mmol) über 10 min zugegeben und anschließend 1 h gerührt. Zu dieser Lösung werden 6 mmol Bis-(3,5-di-methyl-phenyl)chlorphosphan tropfenweise zugegeben und die Reaktionslösung über Nacht auf R.T. erwärmt. Anschließend wird mit Wasserstoffperoxid-Lösung hydrolysiert, weitere 2h gerührt, die Lösung mit Dichlormethan verdünnt und die wäßrige Phase zweimal mit Dichlormethan extrahiert. Nach dem Trocknen und Entfernen des Lösungsmittels, wird das Rohprodukt mittels Chromatographie gereinigt und aus kaltem Pentan kristallisiert. Das Produkt wird in 59% Ausbeute erhalten.
¹H NMR (CDCl₃):
δ = 7.30-7.38 (m, 3H), 7.10-7.22 (m, 3H), 3.93 (dt, J = 11, 5 Hz, 1 H), 2.50-2.60 (m, 1H), 2.48 (s, 3H), 2.37 (s, 12H), 2.22 (s, 3H), 1.07-1.80 (m, 5H), 0.83-0.96 (m, 1H), 0.80 (s, 9H), 0.65-0.77 (m, 1 H), 0.50-0.60 (m, 1 H), 0.00 (s, 3H), -0.13 (s, 3H) ppm.

### Beispiel 22

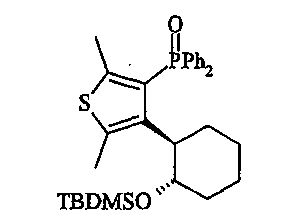

Die Synthese erfolgt analog zu Beispiel 21
Ausbeute: 55%
¹H NMR (d⁶-DMSO):
δ = 7.77-7.88 (m, 10H), 4.10 (dt, J = 10, 4 Hz, 1 H), 2.84-2.93 (m, 1 H), 2.60 (s, 3H), 2.25 (s, 3H), 1.90-1.99 (m, 1H), 1.28-1.70 (m, 6H), 0.91 (s, 9H), 0.54-0.69 (m, 1 H), 0.16 (s, 3H), 0.00 (s, 3H) ppm.

### Beispiel 23

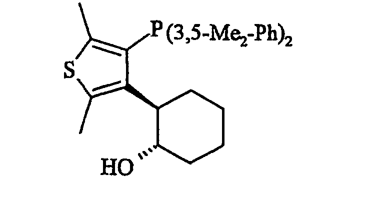

Eine Lösung von 2.89 mmol des Phosphinoxids (Beispiel22) und 28.9 mmol Trichlorsilan in 10 ml Toluol werden 3h unter Rückfluß erhitzt und anschließend auf R.T. abgekühlt. Die Reaktionsmischung wird mit 30%iger Natriumhydroxid-Lösung hydrolysiert, 30 min bei R.T. gerührt und mit 30 ml Wasser verdünnt. Die wäßrige Phase zweimal mit TBME extrahiert. Nach dem Trocknen und Entfernen des Lösungsmittels, wird das Rohprodukt mittels Chromatographie gereinigt. Das Produkt wird in 77% Ausbeute erhalten.
¹H NMR (d⁶-DMSO):
δ = 6.90-7.00 (m, 6H), 4.09-4.23 (m, 1 H), 3.76 (br s, 1H), 2.95-3.08 (m, 1 H), 2.43 (s, 3H), 2.30 (s, 6H), 2.28 (s, 6H), 1.92-2.00 (m, 1 H), 1.90 (s, 3H), 0.96-1.70 (m, 7H) ppm.
³¹P NMR (d⁶-DMSO):
δ = -24.8, -26.1 ppm.

### Beispiel 24

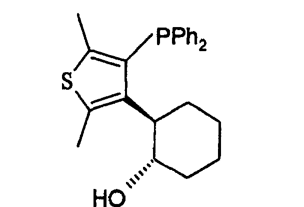

Die Synthese erfolgt analog zu Beispiel 23
Ausbeute: 69%
¹H NMR (d⁶-DMSO):
δ = 7.27-7.40 (m, 10H), 4.10-4.27 (m, 1 H), 3.80 (br s, 1 H), 3.00-3.10 (m, 1 H), 2.42 (s, 3H), 1.90-2.00 (m, 1H), 1.78 (s, 3H), 1.00-1.70 (m, 7H) ppm.
³¹P NMR (d⁶-DMSO):
δ = -24.4, -26.0 ppm.

### Beispiel 25

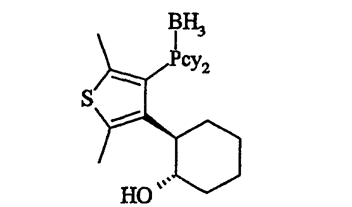

Die Synthese erfolgt analog zu Beispiel 12
Ausbeute: 27%
¹H NMR (C₆H₆):
δ = 3.73-3.83 (m, 1 H), 3.28 (br s, 1 H), 2.70 (br s, 3H), 2.13-2.27 (m, 1 H), 2.10 (s, 3H), 2.00-2.06 (m, 1 H), 1.93 (s, 3H), 1.80-1.87 (m, 1 H), 0.95-1.73 (m, 27H) ppm.
³¹P NMR (C₆H₆):
δ = 36.7 ppm.

### Beispiel 26

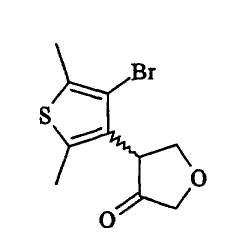

Eine Lösung von 92.4 mmol Oxalylchlorid in 400 ml Dichlormethan wird auf -50°C abgekühlt und 185 mmol DMSO werden innerhalb von 5 min zugegeben. Nach 5 min werden 77 mmol des Alkohols (Beispiel 5) gelöst in 100 ml Dichlormethan zugegeben. Die Lösung wird weitere 30 min gerührt und anschließend werden 385 mmol Triethylamin zugegeben und die Reaktionslösung auf R.T. erwärmt. Die organische Phase wird mit 200 ml Wasser, mit 200 ml 3M Salzsäure und 200 ml Natriumhydrogencarbonat-Lösung gewaschen. Die organische Phase wird getrocknet, das Lösungsmittel im Vakuum entfernt und das Rohprodukt in die nächste Stufe eingesetzt.
¹H NMR (CDCl₃):
δ = 4.44 (t, J = 12 Hz, 1 H), 4.20 (t, J = 11 Hz, 1 H), 4.13 (d, J = 12 Hz, 2H), 3.86 (t, J = 11 Hz, 1 H), 2.27 (s, 3H), 2.26 (s, 3H) ppm.

### Beispiel 27

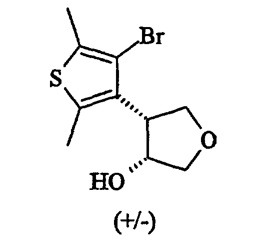

Es werden 77 mmol des Keton (Beispiel 26) in 200 ml THF gelöst. Bei -78°C werden 100 mmol K-Selectrid zugegeben und die Lösung über Nacht auf R.T. erwärmt. Zu dieser Lösung werden 150 ml Ethanol und nacheinander 150 ml 4N Natronlauge und 30%ige Wasserstoffperoxid gegeben. Die Lösung wird weitere 2h gerührt, mit 250 ml TBME verdünnt und die Phase getrennt. Die wäßrige Phase wird zweimal mit 100 ml TBME extrahiert, anschließend wird die org. Phase getrocknet und das Lösungsmittel im Vakuum entfernt. Das Rohprodukt wird aus Heptan umkristallisiert.
Ausbeute: 69%
¹H NMR (CDCl₃):
δ = 4.65-4.70 (m, 1 H), 4.56 (t, J = 11 Hz, 1 H), 4.23 (dd, J = 12, 5 Hz, 1 H), 4.18 (t, J = 10 Hz, 1 H), 4.11 (d, J = 12 Hz, 1 H), 3.82-3.90 (m, 1 H), 2.68 (s, 3H), 2.52 (s, 3H), 1.78 (br s, 1 H) ppm.

### Beispiel 28

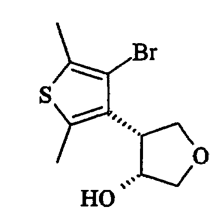

Die Synthese erfolgt analog zu Beispiel 5 unter Verwendung von ChiroCLEC PC
¹H NMR (CDCl₃):
δ = 5.60-5.64 (m, 1 H), 4.54 (t, J = 10 Hz, 1 H), 4.26 (dd, J = 12, 5 Hz, 1 H), 4.10-4.21 (m, 2H), 3.98-4.06 (m, 1 H), 2.63 (m, 3H), 2.50 (m, 3H), 2.03 (m, 3H) ppm.

### Beispiel 29

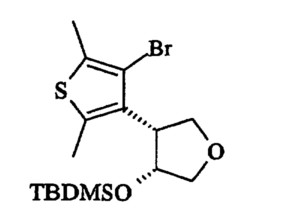

Die Synthese erfolgt analog zu Beispiel 6
Ausbeute: 95%
¹H NMR (CDCl₃):
δ = 4.85 (dt, J = 6, 2 Hz, 1 H), 4.67 (dd, J = 13, 11 Hz, 1 H), 4.36 (dd, J = 13, 6 Hz, 1H), 4.26 (t, J = 11 Hz, 1 H), 4.08 (dd, J = 10, 2 Hz, 1 H), 3.78-3.96 (m, 1 H), 2.70 (s, 3H), 2.60 (s, 3H), 1.00 (s, 9H), 0.11 (s, 3H), 0.00 (s, 3H) ppm.

### Beispiel 30

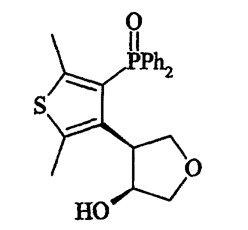

Die Synthese erfolgt analog zu Beispiel 21 und Beispiel 12
Ausbeute: 37%
¹H NMR (C₆H₆):
δ = 7.80-7.94 (m, 4H), 7.18-7.31 (m, 6H), 4.93 (q, J = 8 Hz, 1 H), 4.62 (dd, J = 11, 8 Hz, 1 H), 4.40-4.45 (m, 1 H), 4.31 (dd, J = 11, 8 Hz, 1 H), 4.25 (q, J = 8 Hz, 1 H), 3.97 (t, J = 11 Hz, 1 H), 2.62 (s, 3H), 1.79 (s, 3H) ppm.
³¹P NMR (C₆H₆):
δ = 40.4 ppm.

### Beispiel 31

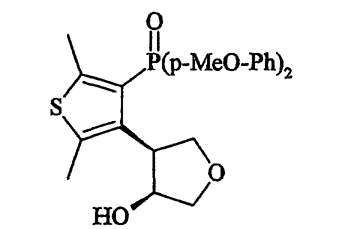

Die Synthese erfolgt analog zu Beispiel 21 und Beispiel 12
Ausbeute: 35%
¹H NMR (C₆H₆):
δ = 7.60-7.73 (m, 4H), 6.68-6.73 (m, 4H), 6.14 (br s, 1 H), 4.85 (q, J = 7 Hz, 1 H), 4.58 (dd, J = 9, 7 Hz, 1 H), 4.30 (dd, J = 10, 8 Hz, 1 H), 4.14-4.23 (m, 2H), 3.89 (t, J = 9 Hz, 1 H), 3.23 (s, 3H), 3.22 (s, 3H), 2.47 (s, 3H), 1.82 (s, 3H) ppm.
³¹P NMR (C₆H₆):
δ = 40.3 ppm.

### Beispiel 32

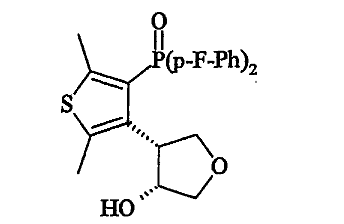

Die Synthese erfolgt analog zu Beispiel 21 und Beispiel 12
Ausbeute: 40%
¹H NMR (CDCl₃):
δ = 7.42-7.52 (m, 4H), 7.04-7.12 (m, 4H), 4.23 (q, J = 7 Hz, 1 H), 4.10 (dd, J = 9, 8 Hz, 1H), 3.96 (dd, J = 10, 7 Hz, 1 H), 3.63-3.70 (m, 2H), 3.58 (t, J = 9 Hz, 1 H), 2.40 (s, 3H), 2.23 (br s, 1H), 1.59 (s, 3H) ppm.
³¹P NMR (CDCl₃):
δ = 27.3 ppm.

### Beispiel 33

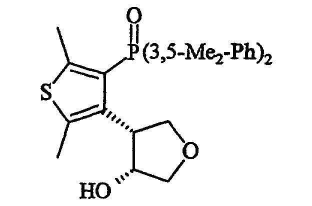

Die Synthese erfolgt analog zu Beispiel 21 und Beispiel 12
Ausbeute: 45%
¹H NMR (CDCl₃):
δ = 7.06-7.14 (m, 6H), 4.20 (q, J = 8 Hz, 1 H), 4.12 (dd, J = 10, 11 Hz, 1 H), 3.96 (dd, J = 11, 9 Hz, 1 H), 3.65-3.76 (m, 2H), 3.60 (t, J = 10 Hz, 1H), 2.45 (s, 3H), 2.28 (s, 6H), 2.27 (s, 6H), 1.61 (s, 3H) ppm.
³¹P NMR (CDCl₃):
δ = 30.0 ppm.

### Beispiel 34

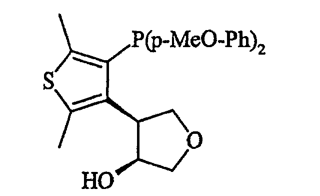

Die Synthese erfolgt analog zu Beispiel 23
Ausbeute: 43%
¹H NMR (C₆H₆):
δ = 7.48-7.55 (m, 4H), 6.94 (d, J = 9 Hz, 2H), 6.88 (d, J = 9 Hz, 2H), 4.52 (t, J = 9 Hz, 1H), 4.21-4.30 (m, 1 H), 4.05-4.10 (m, 1 H), 4.00 (t, J = 9 Hz, 1 H), 3.77-3.80 (m, 2H), 3.45 (s, 3H), 3.43 (s, 3H), 2.62 (s, 3H), 2.40 (s, 3H), 1.66 (br s, 1 H) ppm.
³¹P NMR (C₆H₆):
δ = -11.5 ppm.

### Beispiel 35

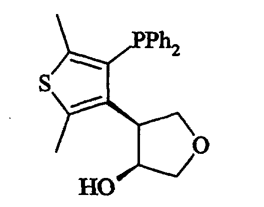

Die Synthese erfolgt analog zu Beispiel 23
Ausbeute: 51 %
¹H NMR (C₆H₆):
δ = 7.43-7.50 (m, 4H), 7.03-7.20 (m, 6H), 4.35 (t, J = 8 Hz, 1 H), 3.90-3.98 (m, 2H), 3.82 (t, J = 9 Hz, 1 H), 3.60 (dd, J = 11, 5 Hz, 1 H), 3.32-3.37 (m, 1 H), 2.52 (s, 3H), 2.35 (s, 3H), 1.33 (br s, 1 H) ppm.
³¹P NMR (C₆H₆):
δ = -11.6 ppm.

### Beispiel 36

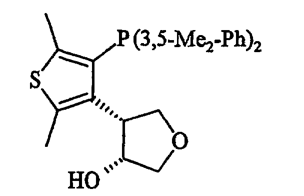

Die Synthese erfolgt analog zu Beispiel 23
Ausbeute: 56%
¹H NMR (C₆H₆):
δ = 7.33 (d, J = 9 Hz, 4H), 6.86 (s, 1 H), 6.80 (s, 1 H), 4.49 (t, J = 8 Hz, 1H), 4.12-4.21 (m, 1 H), 3.98-4.03 (m, 2H), 3.68 (dd, J = 11, 5 Hz, 1 H), 3.49-3.54 (m, 1 H), 2.58 (s, 6H), 2.19 (s, 6H), 2.12 (s, 6H), 1.56 (br s, 1H) ppm.
³¹P NMR (C₆H₆):
δ = -12.1 ppm.

### Beispiel 37

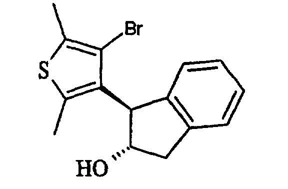

Die Synthese erfolgt analog zu Beispiel 4 und Beispiel 5 unter Verwendung von ChiroCLEC PC
¹H NMR (d⁶-DMSO):
δ = 7.05-7.23 (m, 3H), 6.76 (d, J = 9 Hz, 1 H), 4.68-4.76 (m, 1 H), 4.47 (d, J = 7 Hz, 1 H), 3.28 (dd, J = 18, 9 Hz, 1 H), 3.18 (br s, 1 H), 2.90 (dd, J = 8, 18 Hz, 1H), 2.31 (s, 3H), 2.12 (s, 3H) ppm.

### Beispiel 38

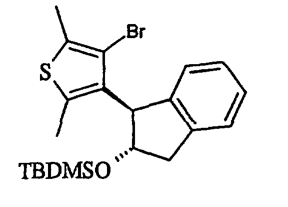

Die Synthese erfolgt analog zu Beispiel 6
Ausbeute: 94 %
¹H NMR (CDCl₃):
δ = 7.17-7.30 (m, 3H), 6.88-7.03 (m, 1H), 5.09-5.18 (m, 1 H^{a}), 4.69-4.87 (m, 2H^{b}), 4.57 (d, J = 8 Hz, 1H^{a}), 3.28-3.43 (m, 1 H), 3.08 (dd, J = 9, 16 Hz, 1 H), 2.56 (s, 3H^{a}), 2.50 (s, 3H^{b}), 2.40 (s, 3H^{a}), 2.00 (s, 3H^{b}), 0.95 (s, 9H), 0.00 (s, 3H), -0.06 (s, 3H) ppm.

### Beispiel 39

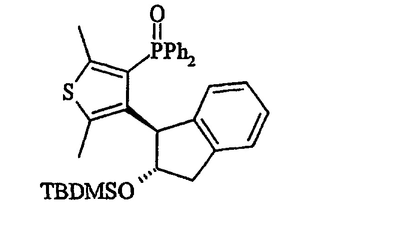

Die Synthese erfolgt analog zu Beispiel 21
Ausbeute: 74%
¹H NMR (d⁶-DMSO):
δ = 7.69-7.87 (m, 10H), 7.36 (d, J = 8 Hz, 1 H), 7.30 (t, J = 9 Hz, 1 H), 7.23 (t, J = 8 Hz, 1H), 6.77 (d, J = 9 Hz, 1 H), 5.60 (d, J = 9 Hz, 1 H), 5.00 (q, J = 9 Hz, 1 H), 3.40 (dd, J = 9, 17 Hz, 1 H), 2.84 (dd, J = 9, 17 Hz, 1 H), 2.08 (s, 3H), 2.02 (s, 3H), 0.98 (s, 9H), 0.06 (s, 3H), 0.00 (s, 3H) ppm.

### Beispiel 40

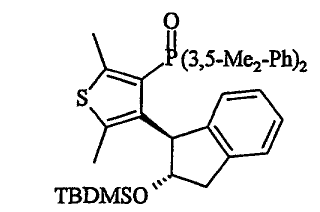

Die Synthese erfolgt analog zu Beispiel 21
Ausbeute: 65%
¹H NMR (d⁶-DMSO):
δ = 7.28-7.44 (m, 9H), 6.86-6.90 (m, 1 H), 5.20 (d, J = 8 Hz, 1 H), 4.96 (q, J = 9 Hz,
1 H), 3.39 (dd, J = 8, 15 Hz, 1 H), 2.75 (dd, J = 10, 15 Hz, 1 H), 2.50 (s, 6H), 2.46 (s, 6H), 2.12 (s, 3H), 2.08 (s, 3H), 1.00 (s, 9H), 0.05 (s, 3H), 0.00 (s, 3H) ppm.

### Beispiel 41

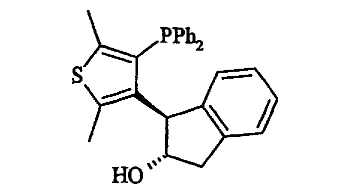

Die Synthese erfolgt analog zu Beispiel 23
Ausbeute: 77%
¹H NMR (d⁶-DMSO):
δ = 6.80-7.40 (m, 13H), 6.60 (d, J = 8 Hz, 1 H), 4.60-5.00 (m, 2H), 3.30 (dd, J = 8, 16 Hz, 1H), 2.85 (dd, J = 7, 16 Hz, 1H), 2.10 (br s, 3H), 1.80 (s, 3H) ppm.
³¹P NMR (d⁶-DMSO):
δ = -23.4, -26.9 ppm.

### Beispiel 42

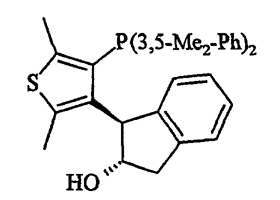

Die Synthese erfolgt analog zu Beispiel 23
Ausbeute: 77%
¹H NMR (d⁶-DMSO):
δ = 7.13 (d, J = 8 Hz, 1 H), 7.05 (t, J = 8 Hz, 1H), 6.71-6.95 (m, 7H), 6.55 (d, J = 9 Hz, 1 H), 4.58-4.90 (m, 2H), 3.32 (dd, J = 9, 17 Hz, 1 H), 2.81 (dd, J = 8, 17 Hz, 1 H), 2.25 (s, 6H), 2.21 (s, 6H), 2.10 (br s, 3H), 1.90 (s, 3H) ppm.
³¹P NMR (d⁶-DMSO):
δ = -23.0, -26.7 ppm.

### Beispiel 43

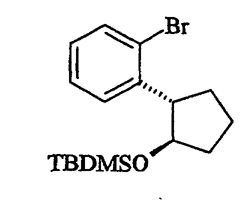

Die Synthese erfolgt analog zu Beispiel 7
Ausbeute: 96%
¹H NMR (CDCl₃):
δ = 7.69 (d, J = 9 Hz, 1 H), 7.40-7.43 (m, 1 H), 7.35 (dd, J = 9, 2 Hz, 1 H), 7.18 (dt, J = 8, 2 Hz, 1 H), 4.38 (q, J = 7 Hz, 1 H), 3.67 (q, J = 8 Hz, 1 H), 2.33-2.42 (m, 1 H), 1.83-2.19 (m, 5H), 0.96 (s, 9H), 0.03 (s, 3H), 0.00 (s, 3H) ppm.

### Beispiel 44

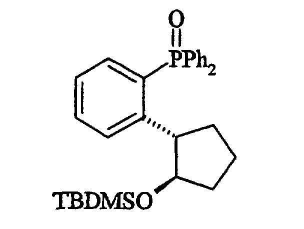

Die Synthese erfolgt analog zu Beispiel 21
Ausbeute: 56%
¹H NMR (C₆H₆):
δ = 7.77-7.83 (m, 2H), 7.66-7.72 (m, 2H), 7.13-7.20 (m, 1 H), 6.93-7.08 (m, 8H), 6.72-6.78 (m, 1 H), 4.35 (q, J = 8 Hz, 1 H), 4.22-4.31 (m, 1 H), 2.22-2.33 (m, 1 H), 1.20-1.82 (m, 5H), 0.92 (s, 9H), 0.00 (s, 6H) ppm.
³¹P NMR (C₆H₆):
δ = 43.4 ppm.

### Beispiel 45

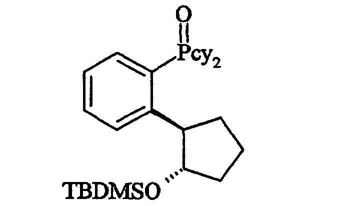

Die Synthese erfolgt analog zu Beispiel 21
Ausbeute: 56%
¹H NMR (C₆H₆):
δ = 7.09-7.20 (m, 3H), 6.91 (t, J = 8 Hz, 1 H), 4.70-4.81 (m, 1 H), 4.30 (q, J = 9 Hz, 1 H), 2.50-2.61 (m, 1H), 2.16-2.25 (m, 1 H), 0.95-2.00 (m, 26H), 0.87 (s, 9H), 0.00 (s, 3H), -0.09 (s, 3H) ppm.
³¹P NMR (C₆H₆):
δ = 62.8 ppm.

### Beispiel 46

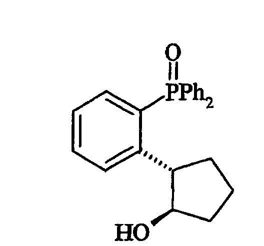

Die Synthese erfolgt analog zu Beispiel 12
Ausbeute: 67%
¹H NMR (C₆H₆):
δ = 7.62 (m, 4H), 7.21-7.29 (m, 1 H), 6.96-7.12 (m, 8H), 6.79 (t, J = 8 Hz, 1 H), 6.46 (br s, 1 H), 4.32 (q, J = 8 Hz, 1 H), 4.05 (q, J = 10 Hz, 1 H), 1.95-2.12 (m, 2H), 1.55-1.77 (m, 4H) ppm.
³¹P NMR (C₆H₆):
δ = 48.2 ppm.

### Beispiel 47

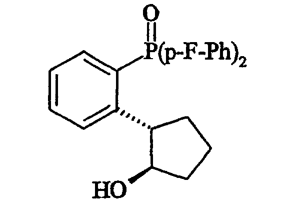

Die Synthese erfolgt analog zu Beispiel 21
Ausbeute: 45%
¹H NMR (C₆H₆):
δ = 7.38-7.49 (m, 4H), 7.25-7.31 (m, 2H), 6.86-6.92 (m, 2H), 6.63-6.75 (m, 4H), 6.30 (br s, 1 H), 4.33 (q, J = 8 Hz, 1 H), 3.90-4.00 (m, 1 H), 1.99-2.17 (m, 2H), 1.60-1.80 (m, 4H) ppm.
³¹P NMR (C₆H₆):
δ = 42.0 ppm.

### Beispiel 48

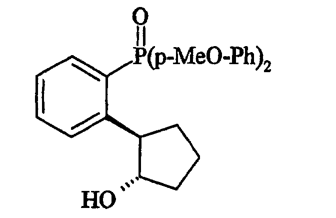

Die Synthese erfolgt analog zu Beispiel 21
Ausbeute: 57%
¹H NMR (C₆H₆):
δ = 7.52-7.62 (m, 4H), 7.21-7.28 (m, 1 H), 7.02-7.18 (m, 2H), 6.81 (t, J = 8 Hz, 1 H), 6.53-6.60 (m, 4H), 4.30 (q, J = 8 Hz, 1 H), 4.10 (q, J = 9 Hz, 1 H), 3.11 (s, 3H), 3.06 (s, 3H), 1.92-2.06 (m, 2H), 1.60-1.80 (m, 2H), 1.25-1.36 (m, 2H) ppm.
³¹P NMR (C₆H₆):
δ = 47.9 ppm.

### Beispiel 49

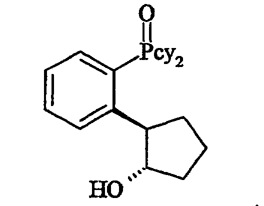

Die Synthese erfolgt analog zu Beispiel 12
Ausbeute: 67%
¹H NMR (C₆H₆):
δ = 7.42 (dd, J = 10, 4 Hz, 1 H), 7.25-7.32 (m, 1 H), 7.01-7.09 (m, 2H), 6.45 (br s, 1 H), 4.76 (q, J = 9 Hz, 1 H), 4.25 (q, J = 7 Hz, 1 H), 2.42-2.50 (m, 1 H), 0.90-2.30 (m, 27H) ppm.
³¹P NMR (C₆H₆):
δ = 66.5 ppm.

### Beispiel 50

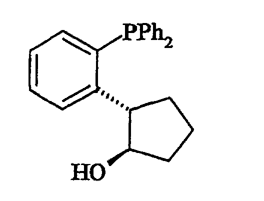

Die Synthese erfolgt analog Beispiel 23
Ausbeute: 57%
¹H NMR (C₆H₆):
δ = 7.54-7.62 (m, 4H), 7.30-7.39 (m, 3H), 7.24-7.29 (m, 6H), 7.15 (dt, J = 9, 2 Hz, 1H), 4.26-4.40 (m, 2H), 2.23-2.32 (m, 1H), 2.02-2.11 (m, 1H), 1.67-1.91 (m, 4H), 1.60 (br s, 1 H) ppm.
³¹P NMR (C₆H₆):
δ = 0.0 ppm.

### Beispiel 51

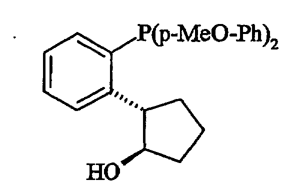

Die Synthese erfolgt analog Beispiel 23
Ausbeute: 37%
¹H NMR (C₆H₆):
δ = 7.40-7.48 (m, 4H), 7.21-7.31 (m, 3H), 7.12 (dt, J = 9, 2 Hz, 1 H), 6.81 (d, J = 11 Hz, 4H), 4.16-4.31 (m, 2H), 3.31 (s, 3H), 3.30 (s, 3H), 2.15-2.25 (m, 1 H), 1.92-2.03 (m, 1H), 1.59-1.85 (m, 4H), 1.56 (br s, 1 H) ppm.
³¹P NMR (C₆H₆):
δ = -5.3 ppm.

### Beispiel 52

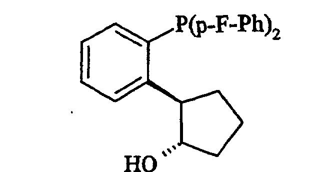

Die Synthese erfolgt analog Beispiel 23
Ausbeute: 60%
¹H NMR (C₆H₆):
δ = 7.00-7.05 (m, 1 H), 6.92-7.03 (m, 5H), 6.80-6.90 (m, 2H), 6.55-6.63 (m, 4H), 4.00 (q, J = 7 Hz, 1 H), 3.85 (quin, J = 8Hz, 1 H), 1.82-1.91 (m, 1 H), 1.69-1.78 (m, 1 H), 1.27-1.60 (m, 4H) ppm.
³¹P NMR (C₆H₆):
δ = -3.0 ppm.

### Beispiel 53

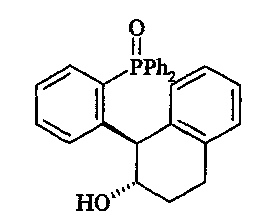

Die Synthese erfolgt analog zu Beispiel 21 und Beispiel 12
Ausbeute: 45%
¹H NMR (C₆H₆):
δ = 7.80-7.94 (m, 4H), 6.91-7.21 (m, 13H), 6.70 (d, J = 9 Hz, 1H), 6.51 (d, J = 9 Hz, 1 H), 5.49 (d, J = 10 Hz, 1 H), 4.32-4.40 (m, 1 H), 2.90-3.00 (m, 1 H), 2.84 (dt, J = 16, 6 Hz, 1 H), 2.50-2.58 (m, 1 H), 2.12-2.23 (m, 1 H) ppm.
³¹P NMR (C₆H₆):
δ = 48.2 ppm.

### Beispiel 54

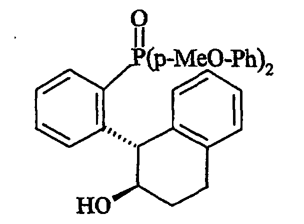

Die Synthese erfolgt analog zu Beispiel 21 und Beispiel 12
Ausbeute: 52%
¹H NMR (C₆H₆):
δ = 7.86-7.99 (m, 4H), 7.38-7.43 (m, 1 H), 7.30-7.36 (m, 2H), 7.00-7.20 (m, 5H), 6.90 (dd, J = 12, 3 Hz, 2H), 6.83 (dd, J = 12, 3 Hz, 2H), 6.70 (d, 9 Hz, 1 H), 5.69 (d, J = 10 Hz, 1H), 4.42-4.51 (m, 1 H), 3.43 (s, 3H), 3.30 (s, 3H), 3.00-3.12 (m, 1 H), 2.94 (dt, J = 16, 4 Hz, 1 H), 2.60-2.69 (m, 1 H), 2.30 (ddd, J = 18, 12, 6 Hz, 1 H) ppm.
³¹P NMR (C₆H₆):
δ = 48.1 ppm.

### Beispiel 55

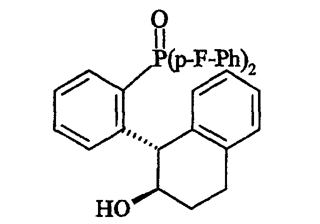

Die Synthese erfolgt analog zu Beispiel 21 und Beispiel 12
Ausbeute: 43%
¹H NMR (C₆H₆):
δ = 7.42-7.50 (m, 2H), 7.32-7.41 (m, 2H), 6.99-7.05 (m, 2H), 6.78-6.94 (m, 4H), 6.63 (dt, J = 9, 3 Hz, 2H), 6.54 (dt, J = 9, 3 Hz, 2H), 6.29 (d, J = 8 Hz, 1 H), 6.25 (d, J = 8 Hz, 1 H), 5.20 (d, J = 8 Hz, 1 H), 4.10-4.20 (m, 1 H), 2.64-2.83 (m, 2H), 2.31-2.39 (m, 1 H), 2.01 (ddd, J = 18, 12, 6 Hz, 1 H) ppm.
³¹P NMR (C₆H₆):
δ = 46.8 ppm.

### Beispiel 56

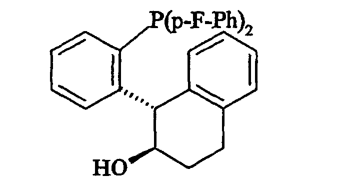

Die Synthese erfolgt analog zu Beispiel 23
Ausbeute: 50%
¹H NMR (C₆H₆):
δ = 7.05-7.15 (m, 5H), 6.85-7.01 (m, 6H), 6.65-6.76 (m, 5H), 5.06-5.20 (m, 1 H), 4.09-4.18 (m, 1 H), 2.68-2.85 (m, 2H), 1.91-2.00 (m, 1 H), 1.63-1.74 (m, 1 H), 1.37 (d, J = 5 Hz, 1 H) ppm.
³¹P NMR (C₆H₆):
δ = -4.1 ppm.

### Beispiel 57

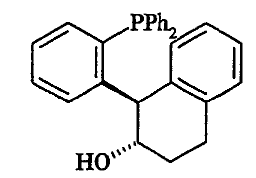

Die Synthese erfolgt analog zu Beispiel 23
Ausbeute: 45%
¹H NMR (C₆H₆):
δ = 7.48-7.55 (m, 5H), 7.32-7.38 (m, 1 H), 7.12-7.20 (m, 6H), 6.99-7.11 (m, 5H), 6.91 (d, J = 7 Hz, 1 H), 5.31-5.41 (m, 1 H), 4.28-4.37 (m, 1 H), 2.78-2.98 (m, 2H), 2.03-2.12 (m, 1 H), 1.73-1.86 (m, 1 H), 1.73 (s, 1H) ppm.
³¹P NMR (C₆H₆):
δ = 0.0 ppm.

### Beispiel 58

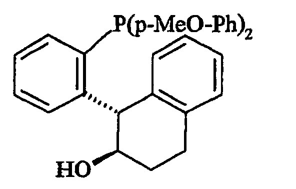

Die Synthese erfolgt analog zu Beispiel 23
Ausbeute: 53%
¹H NMR (C₆H₆):
δ = 7.32-7.40 (m, 4H), 7.25-7.30 (m, 1 H), 6.94-6.99 (m, 4H), 6.86-6.92 (m, 2H), 6.82 (d, J = 7 Hz, 1 H), 6.70 (d, J = 8 Hz, 4H), 5.26-5.33 (m, 1 H), 4.19-4.25 (m, 1H), 3.19 (s, 6H), 2.81 (dt, J = 16, 6 Hz, 1 H), 2.63-2.74 (m, 1 H), 1.96-2.04 (m, 1 H), 1.63-1.77 (m, 2H) ppm.
³¹P NMR (C₆H₆):
δ = -4.4 ppm.

### Beispiel 59 (3R, 4S)-4-(4-{1,1-di[3,5-di(trifluormethyl)phenyl]phosphino]-2,5-dimethyl-3-thienyl)tetrahydro-3-furanyl diphenylphosphinit

Zu einer Lösung 1-(t-Butyl-1,1-dimethylsilyl-((3R,4S)4-(4-(1,1-di(3,5-di(trifluormethyl)phenyl)phosphino)-2,5-dimethyl-3-thienyl)tetrahydro-3-furanyl)ether (0.107 mmol) in Toluol wird unter Eiskühlung Ethylmagnesiumbromid (1 M in THF; 0.102 mmol) zugegeben und 1 h gerührt. Zu dieser Lösung wird im Anschluß Chlordiphenylphosphin (0.107 mmol) zugetropft und über Nacht auf R.T. erwärmt. Nach Filtration über Aluminiumoxid und Entferung des Lösungsmittel wird das Produkt mit 77% Ausbeute isoliert.
¹H NMR (C₆H₆):
δ = 8.11 (d, 2H), 8.06 (d, 2H), 7.9 (s, 2H), 7.77 - 7.68 (m, 4H), 7.38 - 7.24 (m, 6H), 5.50 - 5.40 (m, 1 H), 4.49 - 4.31 (m, 4H), 4.13 (t, 1 H), 2.03 (s, 3H), 1.75 (s, 3H) ppm.
³¹P NMR (C₆H₆)
δ = 127.9 (s, 1 P), -9.9 (s, 1P) ppm.

Die **Beispiele 60 - 89** werden analog zu Beispiel 59 dargestellt.

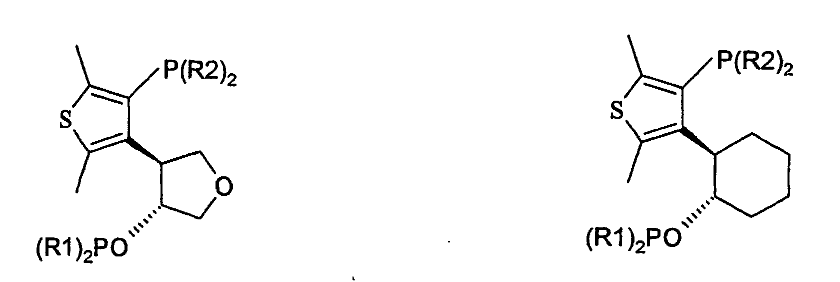

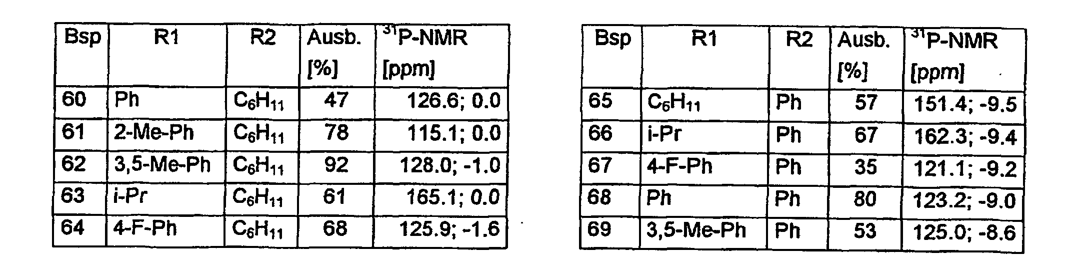

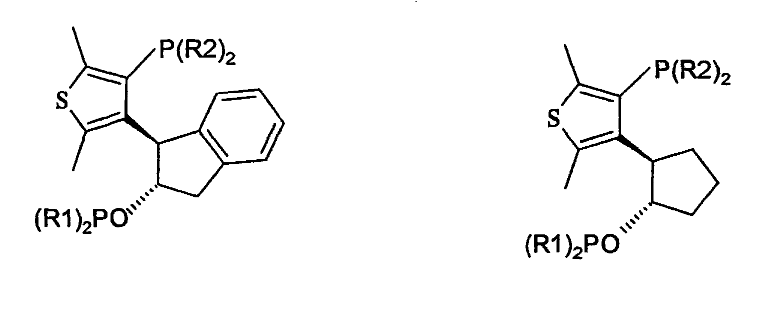

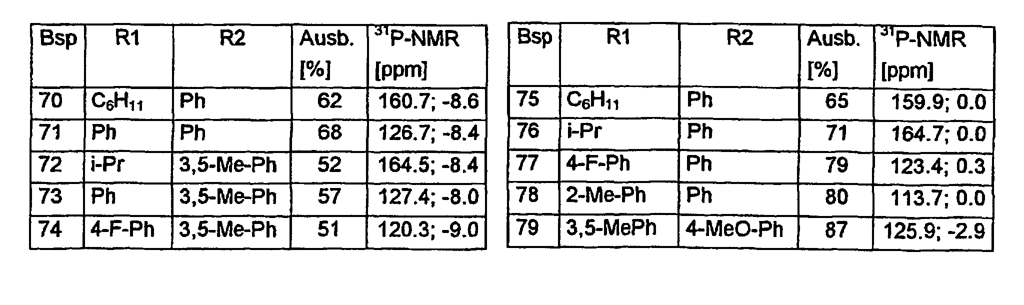

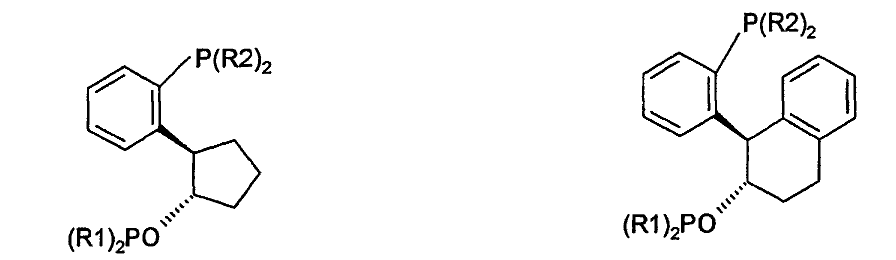

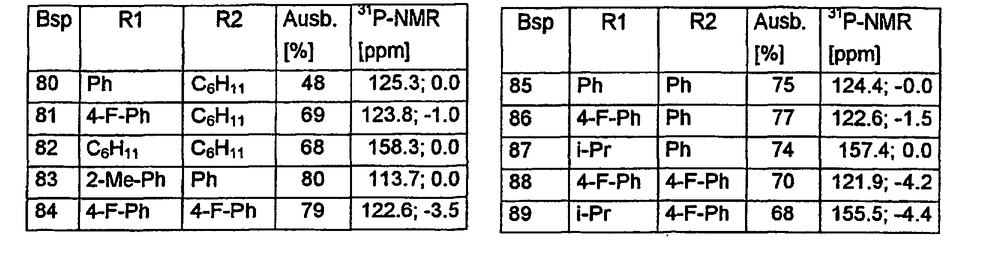

### Beispiel 90: Hydrierungen

### Allgemeine Arbeitsvorschrift zur Hydrierung von Acetamidozimtsäuremethylester

Es werden 0.6 µmol Rh(COD)₂OTf und 0.66 µmol Ligand 10 min in 1 ml Methanol gerührt. Zu dieser Lösung werden 300 µmol Acetamidozimtsäuremethylester (in 1 ml Methanol) dosiert. Bei Raumtemperatur und unter 5 bar Wasserstoffatmosphäre wird die Reaktionsmischung 2h im Autoklaven gerührt. Das Reaktionsgemisch wird über Kieselgel filtriert und aus dem Rohprodukt wird Enantiomerenüberschuß mittels HPLC bestimmt.

### Allgemeine Arbeitsvorschrift zur Hydrierung von Itaconsäuremethylester

Es werden 0.6 µmol Rh(COD)₂OTf und 0.66 µmol Ligand 10 min in 1 ml Methanol gerührt. Zu dieser Lösung werden 300 µmol Itaconsäuremethylester (in 1 ml Methanol) dosiert. Bei 40°C und unter 50 bar Wasserstoffatmosphäre wird die Reaktionsmischung 3h im Autoklaven gerührt. Das Reaktionsgemisch wird über Kieselgel filtriert und aus dem Rohprodukt wird Enantiomerenüberschuß mittels HPLC bestimmt.
HPLC bestimmt.

### Allgemeine Arbeitsvorschrift zur Hydrierung von N-Acetyl-2phenyl-1-ethenylamin

Es werden 0.6 µmol Rh(COD)₂OTf und 0.66 µmol Ligand 10 min in 1 ml Methanol gerührt. Zu dieser Lösung werden 300 µmol N-Acetyl-2phenyl-1-ethenylamin (in 1 ml Methanol) dosiert. Bei 40°C und unter 10 bar Wasserstoffatmosphäre wird die Reaktionsmischung 2h im Autoklaven gerührt. Das Reaktionsgemisch wird über Kieselgel filtriert und aus dem Rohprodukt wird Enantiomerenüberschuß mittels HPLC bestimmt.

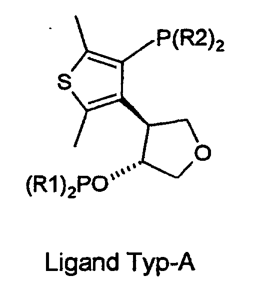

| Ligand Typ-A | | %ee | | |
|---|---|---|---|---|
| R1 | R2 | Acetamidozimtsäuremethylester | Itaconsäuremethylester | N-Acetyl-2phenyl-1-ethenylamin |
| 2-Me-Ph | Ph | 28 | 26 | 46 |
| 2-Me-Ph | 4-F-Ph | 31 | 29 | 15 |
| 3,5-Me-Ph | 4-F-Ph | 3 | 13 | 36 |
| C₆H₁₁ | 4-F-Ph | 24 | -3 | 34 |
| i-Pr | 4-F-Ph | 29 | -11 | 35 |
| 2-Me-Ph | 3,5-Me-Ph | 47 | 13 | 63 |
| 3,5-Me-Ph | 3,5-Me-Ph | 2 | 34 | 57 |
| C₆H₁₁ | 3,5-Me-Ph | 25 | -6 | 34 |
| i-Pr | 3,5-Me-Ph | 6 | -15 | 37 |
| i-Pr | 3,5-CF₃-Ph | -39 | 2 | 41 |
| Ph | 2-Me-Ph | -28 | -6 | -12 |
| 3,5-Me-Ph | C₆H₁₁ | -57 | ― | 27 |

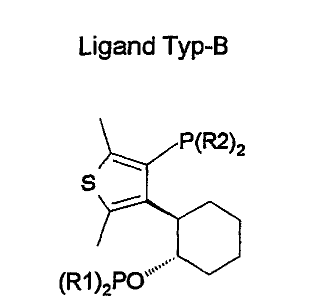

| Ligand Typ-B | | %ee | | |
|---|---|---|---|---|
| R1 | R2 | Acetamidozimtsäuremethylester | Itaconsäuremethylester | N-Acetyl-2phenyl-1-ethenylamin |
| C₆H₁₁ | Ph | 22 | 2 | -1 |
| Ph | Ph | 68 | 8 | 12 |
| 4-F-Ph | Ph | 69 | 21 | 10 |
| 3,5-Me-Ph | Ph | 69 | -3 | 7 |

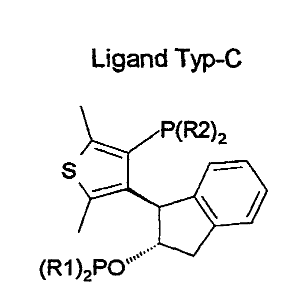

| Ligand Typ-C | | %ee | | |
|---|---|---|---|---|
| R1 | R2 | Acetamidozimtsäuremethylester | Itaconsäuremethylester | N-Acetyl-2phenyl-1-ethenylamin |
| Ph | 3,5-Me-Ph | -14 | 0 | 45 |
| C₆H₁₁ | Ph | 7 | 11 | 34 |
| Ph | Ph | 8 | 2 | 61 |

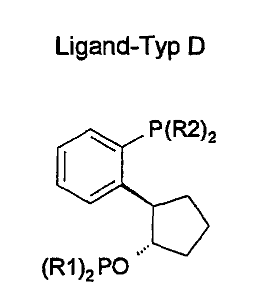

| Ligand-Typ D | | %ee | | |
|---|---|---|---|---|
| R1 | R2 | Acetamidozimtsäuremethylester | Itaconsäuremethylester | N-Acetyl-2phenyl-1-ethenylamin |
| 4-F-Ph | 4-F-Ph | 15 | 3 | 65 |

## Patentansprüche

1. Bidentate Organophosphorliganden der allgemeinen Formeln (Ia) und (Ib), 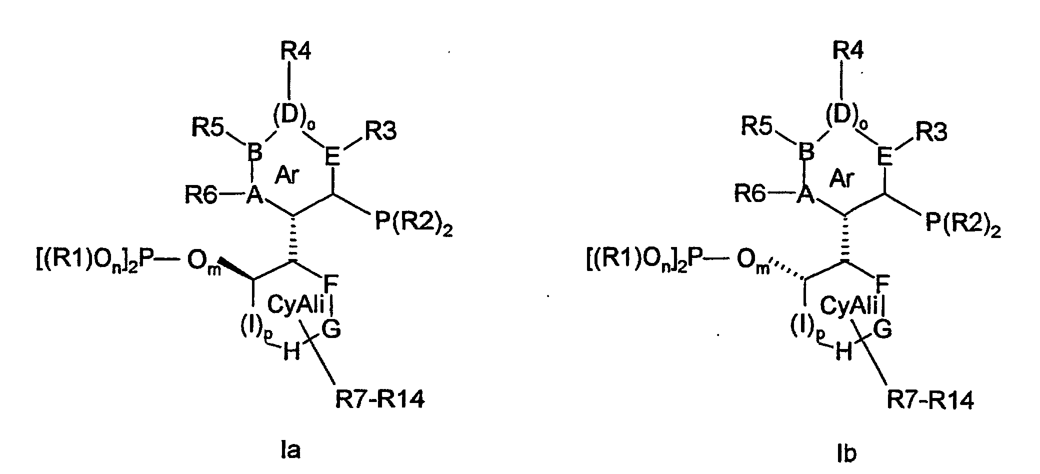 worin
o und p unabhängig voneinander 0 oder 1 sein können und
Ar Teil eines sechsgliedrigen aromatischen oder 5-6 gliedrigen heteroaromatischen Ringsystems sind, wobei das heteroaromatische Ringsystem 1-3 Stickstoffatome, 1 Sauerstoffatom oder 1 Schwefelatom an den Positionen A, B, D und E enthalten kann, und worin
CyAli Teil eines 5-6 gliedrigen cycloaliphatischen oder heterocycloaliphatischen Ringsystems ist, wobei das heterocycloaliphatische Ringsystem 1-2 Heteroatome aus der Gruppe N, O, S an den Positionen F, G, H und I enthalten kann, und worin
R¹-R², unabhängig voneinander C₁-C₂₄ Alkyl, C₃-C₈ Cycloalkyl, wobei der Cyclus auch 1-2 Heteroatome, ausgewählt aus der Gruppe N, O, S, enthalten kann, C₆-C₁₄ Aryl, Phenyl, Naphthyl, Fluorenyl, C₂-C₁₃ Heteroaryl, wobei die Zahl der Heteroatome, ausgewählt aus der Gruppe N, O, S, 1-4 betragen kann, wobei die vorgenannten Gruppen selbst jeweils ein- oder mehrfach unabhängig voneinander mit Wasserstoff, C₁-C₂₀ Alkyl, C₂-C₂₀ Alkenyl, C₁-C₁₀ Haloalkyl, C₃-C₈ Cycloalkyl, C₂-C₉ Heteroalkyl, C₆-C₈ Aryl, Phenyl, Naphthyl, Fluorenyl, C₂-C₆ Heteroaryl, wobei die Zahl der Heteroatome, aus der Gruppe N, O, S, 1-4 betragen kann, C₁-C₁₀ Alkoxy, C₁-C₉ Trihalomethylalkyl, Halogeno, Hydroxy, Trifluormethylsulfonato, Oxo, Thio, Thiolato, Amino, C₁-C₈ substituierte Amino der Formen NH₂, NH-Alkyl-C₁-C₈, NH-Aryl-C₅-C₆, N-Alkyl₂-C₁-C₈, N-Aryl₂-C₅-C₆, N-Alkyl₃-C₁-C₈⁺, N-Aryl₃-C₅-C₆⁺, NH-CO-Alkyl-C₁-C₈, NH-CO-Aryl-C₅-C₆, Cyano, Carboxylato der Formen COOH und COOQ wobei Q entweder ein einwertiges Kation oder C₁-C₈-Alkyl darstellt, C₁-C₆-Acyloxy, Sulfinato, Sulfonato der Formen SO₃H und SO₃Q wobei Q entweder ein einwertiges Kation, C₁-C₈-Alkyl oder C₆-Aryl darstellt, Phosphate der Formen PO₃H₂, PO₃HQ und PO₃Q₂ wobei Q entweder ein einwertiges Kation, C₁-C₈-Alkyl oder C₆-Aryl darstellt, Tri- C₁-C₆ Alkylsilyl ist, wobei R¹ oder R² mit ihrem benachbarten Phosphoratom zu einem 4-8 gliedrigen aliphatischen Zyklus verknüpft sein können, der mit linearen oder verzweigten C₁-C₁₀ alkyl, C₆-aryl, Benzyl, C₁-C₁₀ Alkoxy, Hydroxy oder Benzyloxy, substituiert sein kann, und worin
R³-R¹⁴ unabhängig voneinander ein Wasserstoffatom oder C₁-C₂₄Alkyl, C₁-C₁₀-Haloalkyl, C₃-C₈ Cycloalkyl, C₃-C₈ Cycloalkenyl, wobei der Cyclus auch 1-2 Heteroatome ausgewählt aus der Gruppe N, O, S, enthalten kann, C₆-C₁₄ Aryl, Phenyl, Naphthyl, Fluorenyl, C₂-C₁₃ Heteroaryl, wobei die Zahl der Heteroatome, ausgewählt aus der Gruppe N, O, S, 1-4 betragen kann, wobei die vorgenannten Gruppen selbst jeweils ein - oder mehrfach unabhängig voneinander mit Wasserstoff, C₁-C₂₀ Alkyl, C₂-C₂₀ Alkenyl, C₁-C₁₀ Haloalkyl, C₃-C₈ Cycloalkyl, C₃-C₈ Cycloalkenyl, C₂-C₉ Heteroalkyl, C₁-C₉ Heteroalkenyl, C₆-C₈ Aryl, Phenyl, Naphthyl, Fluorenyl, C₂-C₆ Heteroaryl, wobei die Zahl der Heteroatome, insbesondere aus der Gruppe N, O, S, 1-4 betragen kann, C₁-C₁₀ Alkoxy, C₁-C₉ Trihalomethylalkyl, Trifluormethyl, Trichlormethyl, Fluoro, Chloro, Bromo, lodo, Hydroxy, Trifluormethylsulfonato, Oxo, Thio, Thiolato, Amino, C₁-C₈ substituierte Amino der Formen NH₂, NH-Alkyl-C₁-C₈, NH-Aryl-C₅-C₆, N-Alkyl₂-C₁-C₈, N-Aryl₂-C₅-C₆, N-Alkyl₃-C₁-C₈⁺, N-Aryl₃-C₅-C₆⁺, NH-CO-Alkyl-C₁-C₈, NH-CO-Aryl-C₅-C₆, Cyano, Carboxylato der Formen COOH und COOQ wobei Q entweder ein einwertiges Kation oder C₁-C₈-Alkyl darstellt, C₁-C₆-Acyloxy, Sulfinato, Sulfonato der Formen SO₃H und SO₃Q wobei Q entweder ein einwertiges Kation, C₁-C₈-Alkyl oder C₆-Aryl darstellt, Phosphato der Formen PO₃H₂, PO₃HQ und PO₃Q₂ wobei Q entweder ein einwertiges Kation, C₁-C₈-Alkyl oder C₆-Aryl darstellt, Tri- C₁-C₆ Alkylsilyl substituiert sein können, und wobei zwei dieser Substituenten auch verbrückt sein können, und worin
m, n unabhängig voneinander 1 oder 0 sind, und
P ein dreiwertiger Phosphor ist.

2. Verbindung nach Anspruch 1, **dadurch gekennzeichnet, daß** der sechsgliedrige Heteroaromat ein Pyridyl-Rest oder der fünfgliedrige Heteroaromaten ein Furyl, Thiophenyl oder Pyrrolyl ist und/oder das Cycloaliphatische Ringsystem ein Cyclohexyl-, Cyclopentyl- Rest ist oder als Teil eines annelierten Systems ein Indenyl- und Tetrahydronaphtalinyl- Rest ist oder das heterpaliphatische Ringsystem ein Tetrahydrofuranyl-, Tetrahydrothiophenyl-, Pyrrolidinyl, Piperidinyl- Rest ist

3. Verbindungen nach Anspruch 1 oder 2, **dadurch gekennzeichnet, daß** die Substituenten R1 und R2 unabhängig voneinander für 1-Methylethyl, Cyclohexyl, Cyclopentyl, Phenyl, 2-Methyl-phenyl, 3,5-Dimethyl-phenyl, 4-Methyl-phenyl, 4-Methoxy-phenyl, 3,5-Bis-(trifluormethyl)-phenyl, 4-Trifluormethyl-phenyl, 3,5-Dimethyl-4-Methoxy-phenyl, 4-Phenoxyl, 4-Dialkyamino, 2-Alkylphenyl, 3-Alkylphenyl, 4-Alkylphenyl, 2,6-Dialkylphenyl, 3,5-Dialkylphenyl, 3,4,5-Trialkylphenyl, 2-Alkoxyphenyl, 3-Alkoxyphenyl, 4-Alkoxyphenyl, 2,6-Dialkoxylphenyl, 3,5-Dialkoxyphenyl, 3,4,5-Triialkoxyphenyl, 3,5-Dialkyl-4-Alkoxyphenyl, 3,5-Dialkyl-4-dialkylaminophenyl, 4-Dialkylamino, 3,5-Trifluormethyl, 4-Trifluormethyl, 2-Sulfonyl, 3-Sulfonyl, 4-Sulfonyl, ein bis vierfach halogenierte Phenyl und Naphtyl stehen.

4. Verbindungen nach Anspruch 1, **dadurch gekennzeichnet, daß** die Verbindungen der Formel Ia und Ib enantiomerenangereichert sind.

5. Verbindungen nach Anspruch 4, **dadurch gekennzeichnet, daß** die Enantiomerenanreicherung 90 % übersteigt.

6. Verbindungen nach einem der vorherigen Ansprüche mit einer Struktur 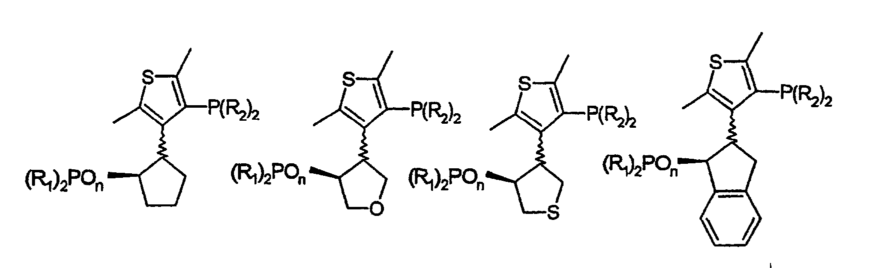 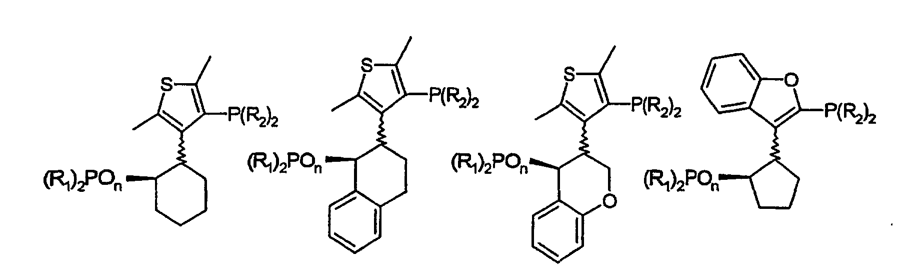 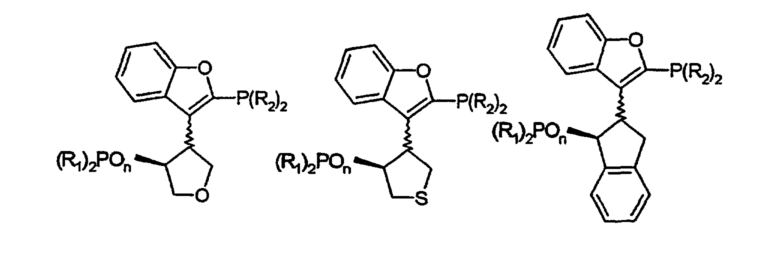 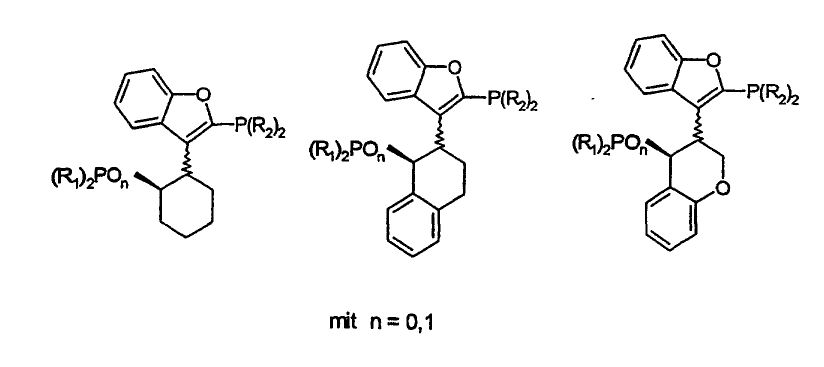 mit X1, X2, X3 = H, OMe, OEt, F, Me, Et n = 0,1 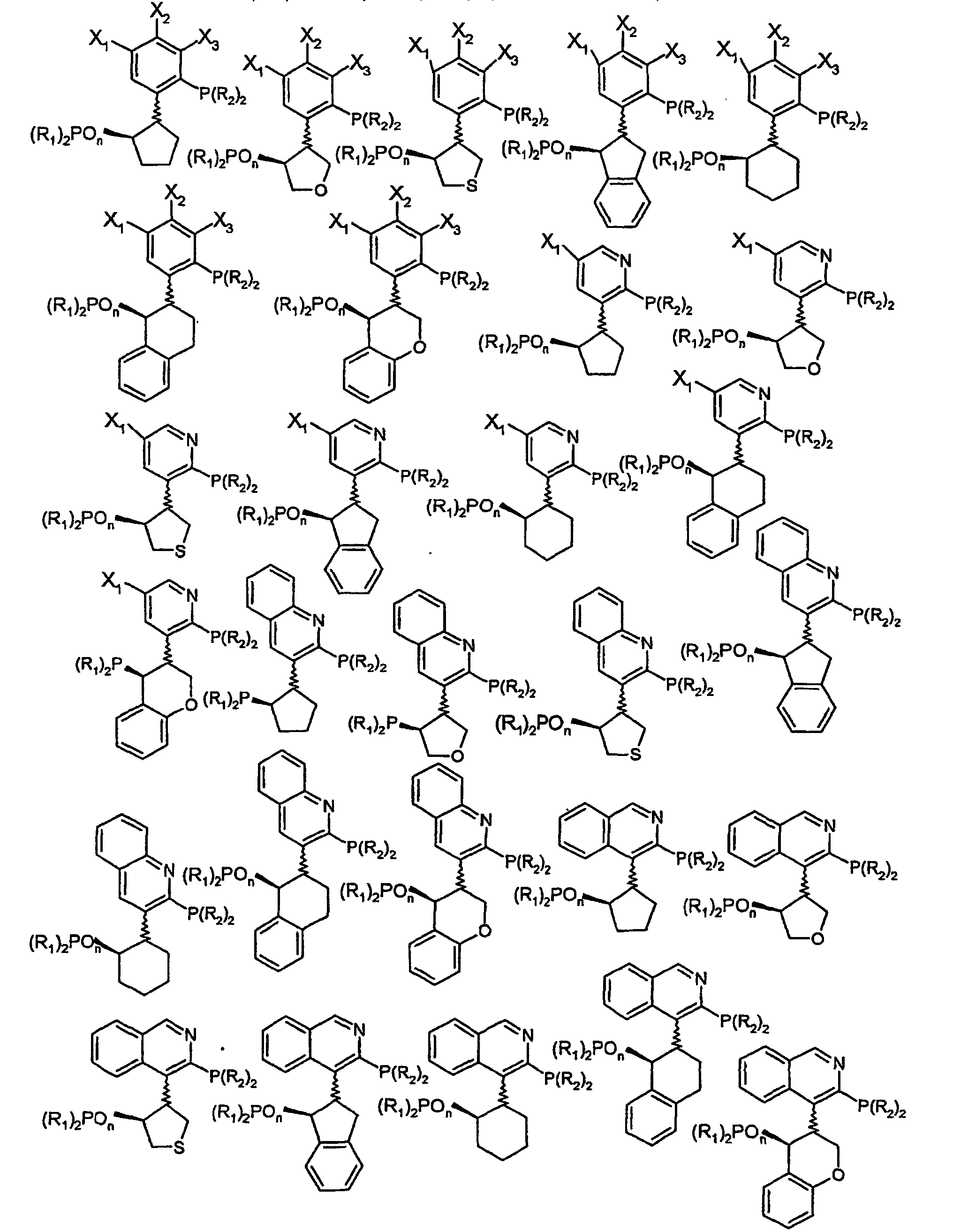 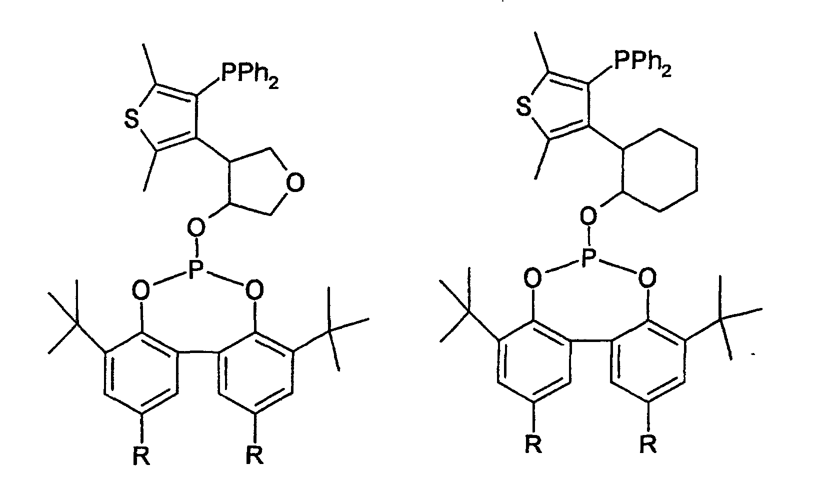 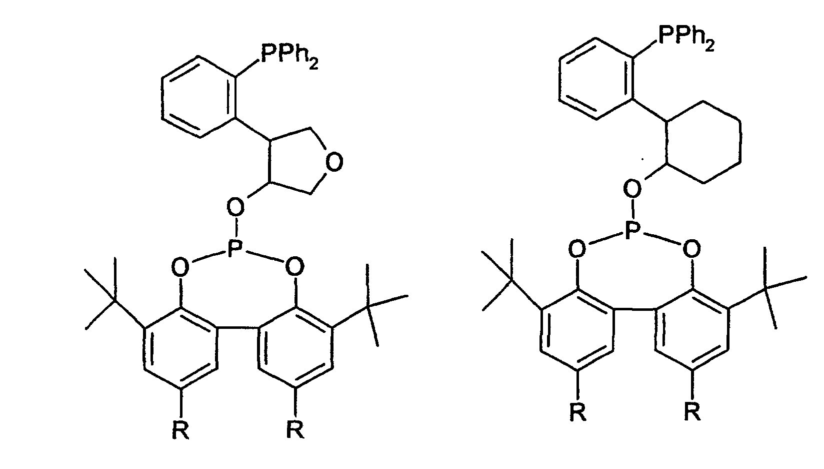 mit R = OMe, t-Butyl

7. Komplexverbindungen der allgemeinen Formel (II)
[MₓP_{y}L_{z}S_{q}]Aᵣ (II)
wobei in der Formel (II)
M ein Metallzentrum
L gleiche oder verschiedene koordinierende organische oder anorganische Liganden,
S koordinierende Lösungsmittelmoleküle und
A Äquivalente aus nicht koordinierenden Anionen repräsentiert,
wobei x und y ganzen Zahlen größer oder gleich 1, z, q und r ganzen Zahlen größer oder gleich 0, die Summe y + z + q wird durch die an den Metallzentren zur Verfügung stehenden Koordinationszentren nach oben begrenzt, wobei nicht alle Koordinationsstellen besetzt sein müssen,
**dadurch gekennzeichnet, daß** P erfindungsgemäße bidentate Organophosphorliganden der Formeln (Ia) und/oder (Ib) ist.

8. Komplexverbindung, die Verbindungen nach Anspruch 7, **dadurch gekennzeichnet, daß** die Verbindungen Palladium, Platin, Rhodium, Ruthenium, Osmium, Iridium, Kobalt, Nickel, oder/und Kupfer enthalten.

9. Verwendung einer Komplexverbindung nach Anspruch 7 oder 8 als Katalysator.

10. Verfahren zur Herstellung von Verbindungen der Formeln (Ia) und (Ib), umfassend folgende Verfahrensschritte:
a) Synthese des aliphatisch-aromatischen Grundgerüstes durch Negishi-Kreuzkuppulng von cyclischen Vinyliodiden mit Halogenaromaten oder durch nucleophile Ringöffnung eines meso-Epoxids mit nachfolgender Racematspaltung,
b) Einführung einer chiralen Phosphineinheit in das Grundgerüst durch assymmetrische Hydroborierung mit einem chiralen Boran, Phospinierung unter Retention oder Inversion des stereogenen Zentrums und
c) Einführung einer zweiten Phosphingruppe durch Brom/Litiumaustausch mit einer starken Litiumbase und nachfolgender Zugabe eines Chlorphosphans.

11. Verfahren zur Herstellung von Biphosphinen nach Anspruch 10, **dadurch gekennzeichnet, daß** zur Phosphinierung unter Retention des stereogenen Zentrums der Hydroborierungsansatz vor der Phospinierung mit Zinkdiorganylen transmetalliert wird.

12. Verfahren zur Herstellung von Biphosphinen nach Anspruch 10, **dadurch gekennzeichnet, daß** zur Phospinierung unter Inversion des stereogenen Zentrums der Hydroborierungsansatz oxidativ unter Bildung des chiralen Alkohols aufgearbeitet und die chirale Hydroxygruppe anschließend geschützt wird, wobei nach Phosphinierung gemäß Anspruch 10 Schritt c) die Hydroxygruppe durch Abspaltung der Schutzgruppe in den Phosphinalkohol überführt und anschließend unter S_{N}2-Bedingungen phosphiniert wird.

13. Verfahren zur Herstellung von Phosphin-Phosphiniten und Phosphin-Phosphiten nach Anspruch 10, **dadurch gekennzeichnet, daß** zur Phosphinierung unter Retention des stereogenen Zentrums der Hydroborierungsansatz oxidativ unter Bildung des chiralen Alkohols aufgearbeitet wird, wobei anschließend in Gegenwart einer Base phophiniert wird.

14. Verfahren zur Herstellung von Phosphin-Phosphiniten und Phosphin-Phosphiten nach Anspruch 13, **dadurch gekennzeichnet, daß** zur Phosphinierung unter Inversion des stereogenen Zentrums der Hydroborierungsansatz oxidativ unter Bildung des chiralen Alkohols aufgearbeitet wird und die Inversion des stereogenen Zentrums durch eine Mitsonubu-Reaktion oder in einem zweistufen Prozeß über eine Oxidation mit anschließender diastereoselektiver Reduktion erfolgt.

15. Verwendung einer Komplexverbindung nach einem der Ansprüche 7 bis 9 als Katalysator für asymmetrisache Reaktionen.

16. Verwendung einer der Komplexverbindungen nach einem der Ansprüche 7 bis 9 als Katalysator für asymmetrischen Hydrierungen, Hydroformulierungen, Umlagerungen, allylische Alkylierungen, Cyclopropenierung, Hydrosilylierungen, Hydrocyanierungen oder Aldolreaktionen.

## Claims

1. Bidentate organophosphorus ligands of the general formulae (Ia) and (Ib) 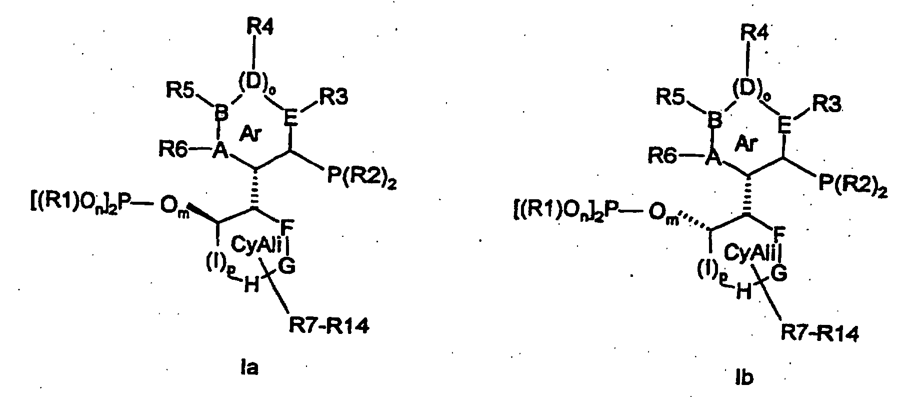 wherein
o and p each independently of the other may be 0 or 1 and
Ar is part of a six-membered aromatic or 5- or 6-membered heteroaromatic ring system, it being possible for the heteroaromatic ring system to contain from 1 to 3 nitrogen atoms, 1 oxygen atom or 1 sulfur atom at positions A, B, D and E, and wherein
CyAli is part of a 5- or 6-membered cycloaliphatic or heterocycloaliphatic ring system, it being possible for the heterocycloaliphatic ring system to contain 1 or 2 hetero atoms from the group N, O, S at positions F, G, H and I, and wherein
R¹-R² each independently of the other is C₁-C₂₄-alkyl, C₃-C₈-cycloalkyl, it being possible for the ring also to contain 1 or 2 hetero atoms selected from the group N, O, S, C₆-C₁₄-aryl, phenyl, naphthyl, fluorenyl, C₂-C₁₃-heteroaryl, it being possible for the number of hetero atoms, selected from the group N, O, S, to be from 1 to 4, wherein the above-mentioned groups themselves in each case one or more times independently of one another by hydrogen, C₁-C₂₀-alkyl, C₂-C₂₀-alkenyl, C₁-C₁₀-haloalkyl, C₃-C₈-cycloalkyl, C₂-C₉-heteroalkyl, C₆-C₈-aryl, phenyl, naphthyl, fluorenyl, C₂-C₆-heteroaryl, it being possible for the number of hetero atoms from the group N, O, S to be from 1 to 4, C₁-C₁₀-alkoxy, C₁-C₉-trihalomethylalkyl, halogen, hydroxy, trifluoromethylsulfonato, oxo, thio, thiolato, amino, C₁-C₈ substituted amino of the forms NH₂, NH-C₁-C₈-alkyl, NH-C₅-C₆-aryl, N-C₁-C₈-alkyl₂, N-C₅-C₆-aryl₂, N-C₁-C₈⁺-alkyl₃, N-C₅-C₆⁺-aryl₃, NH-CO-C₁-C₈-alkyl, NH-CO-C₅-C₆-aryl, cyano, carboxylato of the forms COOH and COOQ (wherein Q represents either a monovalent cation or C₁-C₈-alkyl), C₁-C₆-acyloxy, sulfinato, sulfonato of the forms SO₃H and SO₃Q (wherein Q represents either a monovalent cation, C₁-C₈-alkyl or C₆-aryl), phosphato of the forms PO₃H₂, PO₃HQ and PO₃Q₂ (wherein Q represents either a monovalent cation, C₁-C₈-alkyl or C₆-aryl), tri-C₁-C₆-alkylsilyl, it being possible for R¹ or R² to be linked to its adjacent phosphorus atom to form a 4- to 8-membered aliphatic ring which may be substituted by linear or branched C₁-C₁₀-alkyl, C₆-aryl, benzyl, C₁-C₁₀-alkoxy, hydroxy or benzyloxy, and wherein
R³-R¹⁴ each independently of the others is a hydrogen atom or C₁-C₂₄-alkyl, C₁-C₁₀-haloalkyl, C₃-C₈-cycloalkyl, C₃-C₈-cycloalkenyl, it being possible for the ring also to contain 1 or 2 hetero atoms selected from the group N, O, S, C₆-C₁₄-aryl, phenyl, naphthyl, fluorenyl, C₂-C₁₃-heteroaryl, it being possible for the number of hetero atoms, selected from the group N, O, S, to be from 1 to 4, wherein the above-mentioned groups may themselves be substituted by one or more identical or different substituents selected from hydrogen, C₁-C₂₀-alkyl, C₂-C₂₀-alkenyl, C₁-C₁₀-haloalkyl, C₃-C₈-cycloalkyl, C₃-C₈-cycloalkenyl, C₂-C₉-heteroalkyl, C₁-C₉-heteroalkenyl, C₆-C₈-aryl, phenyl, naphthyl, fluorenyl, C₂-C₆-heteroaryl, it being possible for the number of hetero atoms, especially from the group N, O, S, to be from 1 to 4, C₁-C₁₀-alkoxy, C₁-C₉-trihalomethylalkyl, trifluoromethyl, trichloromethyl, fluoro, chloro, bromo, iodo, hydroxy, trifluoromethylsulfonato, oxo, thio, thiolato, amino, C₁-C₈ substituted amino of the forms NH₂, NH-C₁-C₈-alkyl, NH-C₅-C₆-aryl, N-C₁-C₈-alkyl₂, N-C₅-C₆-aryl₂, N-C₁-C₈⁺-alkyl₃, N-C₅-C₆⁺-aryl₃, NH-CO-C₁-C₈-alkyl, NH-CO-C₅-C₆-aryl, cyano, carboxylato of the forms COOH and COOQ (wherein Q represents either a monovalent cation or C₁-C₈-alkyl), C₁-C₆-acyloxy, sulfinato, sulfonato of the forms SO₃H and SO₃Q (wherein Q represents either a monovalent cation, C₁-C₈-alkyl or C₆-aryl), phosphato of the forms PO₃H₂, PO₃HQ and PO₃Q₂ (wherein Q represents either a monovalent cation, C₁-C₈-alkyl or C₆-aryl), and tri-C₁-C₆-alkylsilyl,
and wherein two of these substituents may also be bridged, and wherein
m,n each independently of the other is 1 or 0, and
P is a trivalent phosphorus.

2. Compound according to claim 1, **characterised in that** the six-membered heteroaromatic ring is a pyridyl radical or the five-membered heteroaromatic ring is a furyl, thiophenyl or pyrrolyl radical and/or the cycloaliphatic ring system is a cyclohexyl, cyclopentyl radical or, as part of a fused system, is an indenyl and tetrahydronaphthalinyl radical, or the heteroaliphatic ring system is a tetrahydrofuranyl, tetrahydrothiophenyl, pyrrolidinyl, piperidinyl radical.

3. Compounds according to claim 1 or 2, **characterised in that** the substituents R1 and R2 each independently of the other is 1-methylethyl, cyclohexyl, cyclopentyl, phenyl, 2-methyl-phenyl, 3,5-dimethyl-phenyl, 4-methyl-phenyl, 4-methoxy-phenyl, 3,5-bis-(trifluoromethyl)-phenyl, 4-trifluoromethyl-phenyl, 3,5-dimethyl-4-methoxy-phenyl, 4-phenoxyl, 4-dialkylamino, 2-alkylphenyl, 3-alkylphenyl, 4-alkylphenyl, 2,6-dialkylphenyl, 3,5-dialkylphenyl, 3,4,5-trialkylphenyl, 2-alkoxyphenyl, 3-alkoxyphenyl, 4-alkoxyphenyl, 2,6-dialkoxyphenyl, 3,5-dialkoxyphenyl, 3,4,5-trialkoxyphenyl, 3,5-dialkyl-4-alkoxyphenyl, 3,5-dialkyl-4-dialkylaminophenyl, 4-dialkylamino, 3,5-trifluoromethyl, 4-trifluoromethyl, 2-sulfonyl, 3-sulfonyl, 4-sulfonyl, mono- to tetra-halogenated phenyl and naphthyl.

4. Compounds according to claim 1, **characterised in that** the compounds of formulae Ia and Ib are enantiomerically enriched.

5. Compounds according to claim 4, **characterised in that** the enantiomeric enrichment exceeds 90 %.

6. Compounds according to any one of the preceding claims having a structure 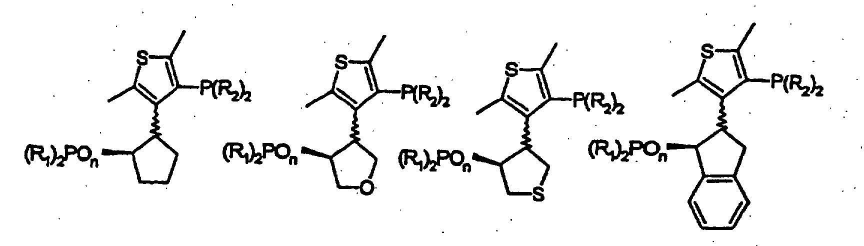 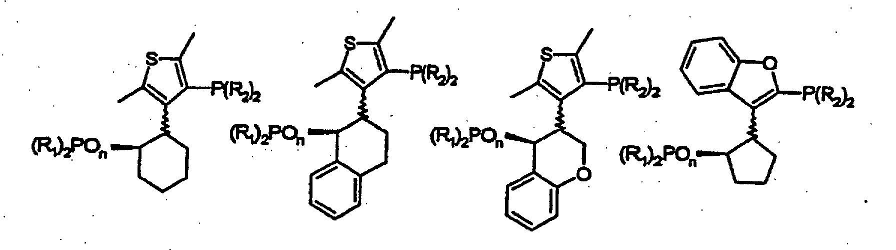 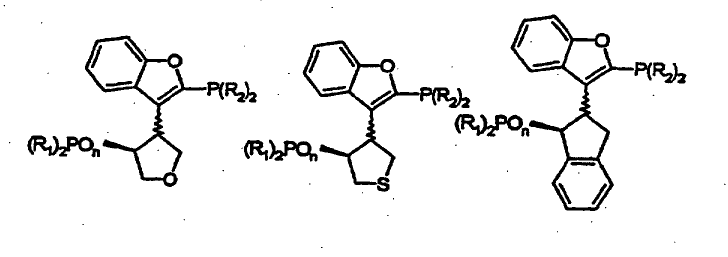 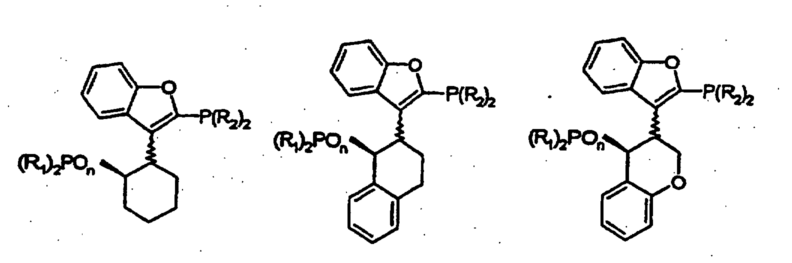 where n = 0, 1
where X1, X2, X3 = H, OMe, OEt, F, Me, Et n = 0, 1 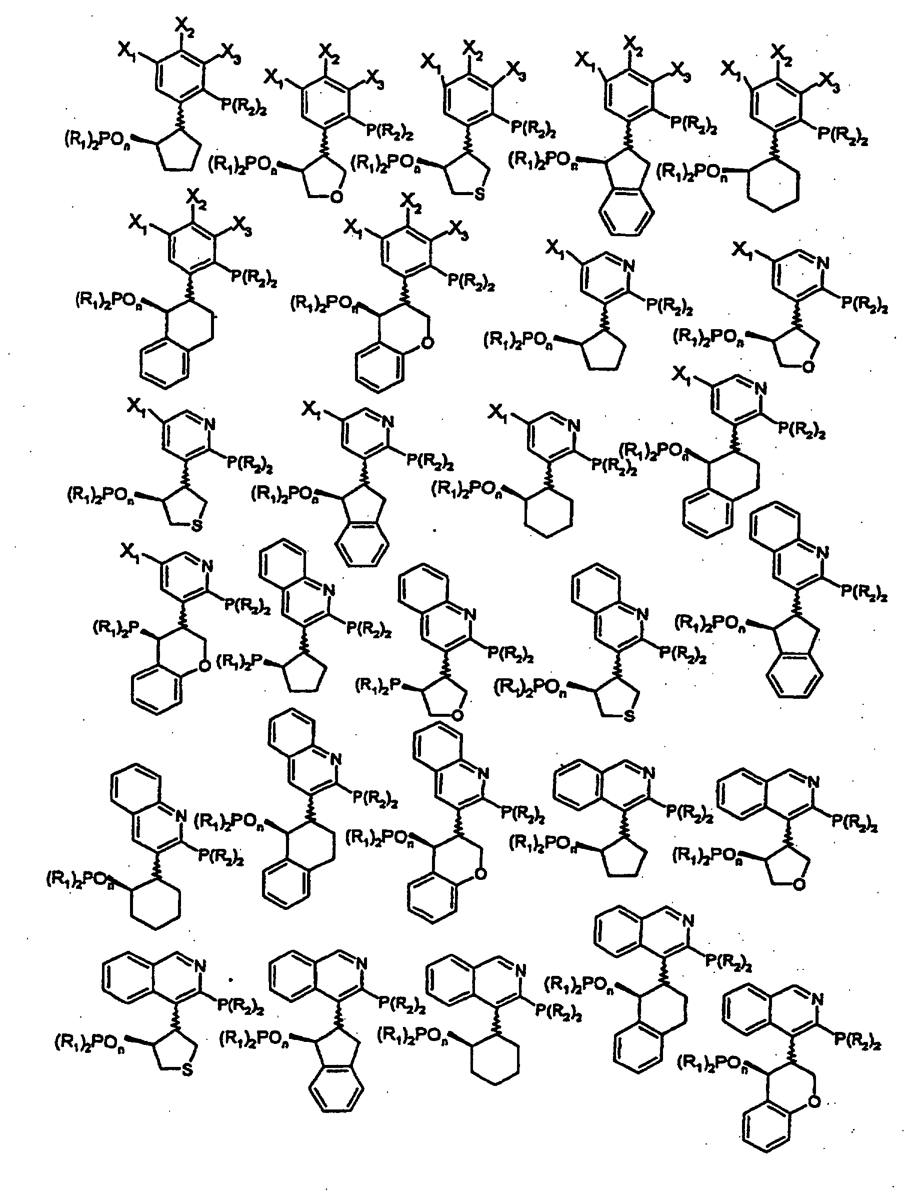 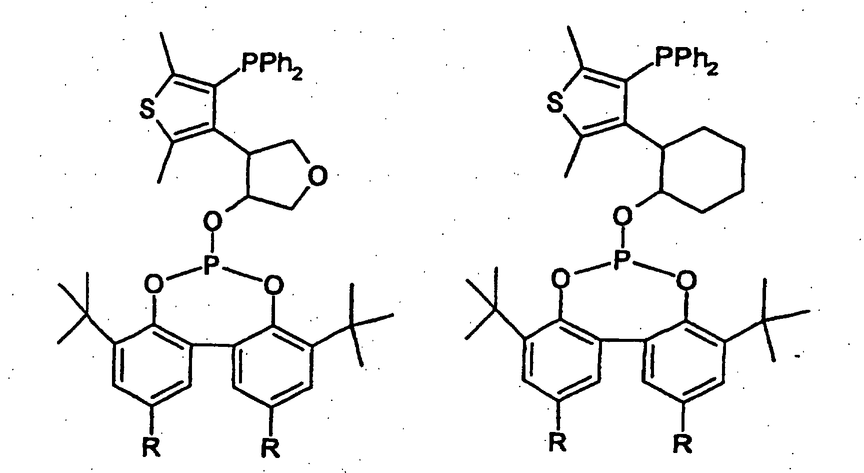 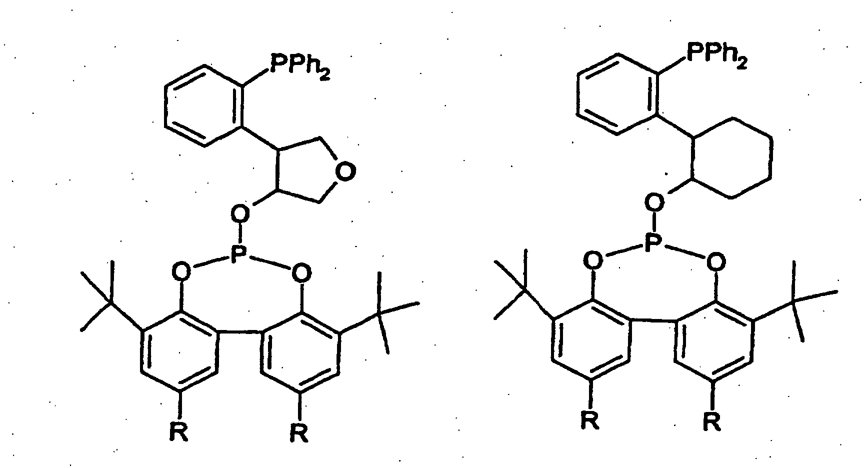 where R = OMe, tert.-butyl

7. Complex compounds of the general formula (II)
[MₓP_{y}L_{z}S_{q}]Aᵣ (II)
wherein in formula (II)
M represents a metal centre,
L represents identical or different coordinating organic or inorganic ligands,
S represents coordinating solvent molecules and
A represents equivalents of non-coordinating anions, wherein x and y are integers greater than or equal to 1, z, q and r are integers greater than or equal to 0, the upper limit of the sum of y + z + q is given by the coordination centres available at the metal centres, not all coordination sites having to be occupied,
**characterised in that** P is bidentate organophosphorus ligands according to the invention of formulae (Ia) and/or (Ib).

8. Complex compound, the compounds according to claim 7, **characterised in that** the compounds comprise palladium, platinum, rhodium, ruthenium, osmium, iridium, cobalt, nickel or/and copper.

9. Use of a complex compound according to claim 7 or 8 as a catalyst.

10. Process for the preparation of compounds of formulae (Ia) and (Ib), comprising the following process steps:
a) synthesis of the aliphatic-aromatic basic structure by Negishi cross-coupling of cyclic vinyl iodides with haloaromatic compounds or by nucleophilic ring opening of a meso-epoxide with subsequent racemate cleavage,
b) introduction of a chiral phosphine unit into the basic structure by asymmetric hydroboronation using a chiral borane, phosphination with retention or inversion of the stereogenic centre, and
c) introduction of a second phosphine group by bromine/lithium substitution using a strong lithium base and subsequent addition of a chlorophosphane.

11. Process for the preparation of biphosphines according to claim 10, **characterised in that**, for phosphination with retention of the stereogenic centre, the hydroboronation batch is transmetallated with zinc diorganyls prior to the phosphination.

12. Process for the preparation of biphosphines according to claim 10, **characterised in that**, for phosphination with inversion of the stereogenic centre, the hydroboronation batch is worked up oxidatively with formation of the chiral alcohol, and the chiral hydroxy group is then protected, wherein, after phosphination according to claim 10 step c), the hydroxy group is converted into the phosphine alcohol by removal of the protecting group and is then phosphined under S_{N}2 conditions.

13. Process for the preparation of phosphine phosphinites and phosphine phosphites according to claim 10, **characterised in that**, for phosphination with retention of the stereogenic centre, the hydroboronation batch is worked up oxidatively with formation of the chiral alcohol, phosphination subsequently being carried out in the presence of a base.

14. Process for the preparation of phosphine phosphinites and phosphine phosphites according to claim 13, **characterised in that**, for phosphination with inversion of the stereogenic centre, the hydroboronation batch is worked up oxidatively with formation of the chiral alcohol and inversion of the stereogenic centre is effected by means of a Mitsonubu reaction or in a two-step process via oxidation with subsequent diastereoselective reduction.

15. Use of a complex compound according to any one of claims 7 to 9 as a catalyst for asymmetric reactions.

16. Use of one of the complex compounds according to any one of claims 7 to 9 as a catalyst for asymmetric hydrogenations, hydroformulations, rearrangements, allylic alkylations, cyclopropenation, hydrosilylations, hydrocyanations or aldol reactions.

## Revendications

1. Ligands organophosphorés bidentates de formules générales (I) et (Ib), 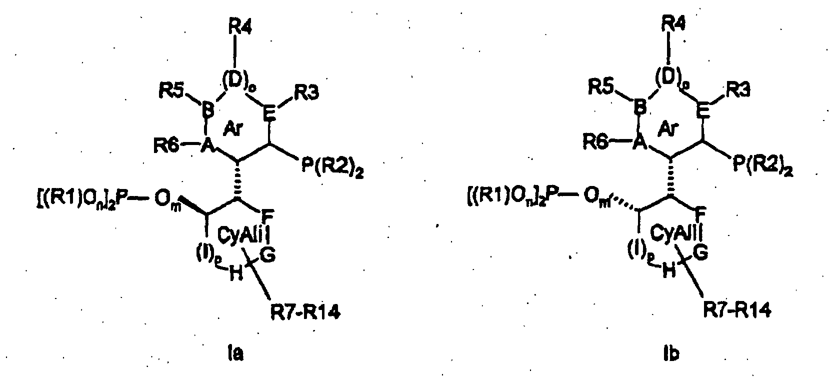 dans lesquelles
o et p peuvent représenter indépendamment l'un de l'autre 0 ou 1 et
Ar est une partie d'un système cyclique aromatique à six membres ou hétéroaromatique à 5 ou 6 membres, le système cyclique hétéroaromatique pouvant contenir 1 à 3 atomes d'azote, 1 atome d'oxygène ou 1 atome de soufre dans les positions A, B, D et E, et dans lesquelles
CyAli est une partie d'un système cyclique cycloaliphatique ou hétérocycloaliphatique à 5 ou 6 membres, le système cyclique hétérocycloaliphatique pouvant contenir 1 à 2 hétéroatomes du groupe N, O, S dans les positions F, G, H et I, et dans lesquelles
R¹ - R² représentent, indépendamment l'un de l'autre, un radical alkyle en C₁ à C₂₄, cycloalkyle en C₃ à C₈, le cycle pouvant également contenir 1 ou 2 hétéroatomes choisis dans le groupe N, O, S, aryle en C₆ à C₁₄, phényle, naphtyle, fluorényle, hétéroaryle en C₂ à C₁₃, le nombre d'hétéroatomes, choisis dans le groupe N, O, S, pouvant être de 1 à 4, les groupes susmentionnés pouvant eux-mêmes être monosubstitués ou polysubstitués, indépendamment l'un de l'autre, par hydrogène, alkyle en C₁ à C₂₀, alcényle en C₂ à C₂₀, halogénoalkyle en C₁ à C₁₀, cycloalkyle en C₃ à C₈, hétéroalkyle en C₂ à C₉, aryle en C₆ à C₈, phényle, naphtyle, fluarényle, hétéroaryle en C₂ à C₆, le nombre d'hétéroatomes du groupe N, O, S pouvant être de 1 à 4, alcoxy en C₁ à C₁₀, trihalogénométhylalkyle en C₁ à C₉, halogèno, hydroxy, trifluorométhylsulfonato, oxo, thio, thiolato, amino, amino substitué en C₁ à C₈ des formes NH₂, NH-(alkyle en C₁ à C₈), NH-(aryle en C₅ à C₆), N-(alkyle en C₁ à C₈)₂, N-(aryle en C₅-C₆)₂, N-(alkyle en C₁ à C₈)₃⁺, N-(aryle en C₅-C₆)₃⁺, NH-CO-(alkyle en C₁ à C₈), NH-CO-(aryle en C₅ à C₆), cyano, carboxylato des formes COOH et COOQ, où Q représente un cation monovalent ou alkyle en C₁ à C₈, acyloxy en C₁ à C₆, sulfinato, sulfonato des formes SO₃H et SO₃Q où Q représente un cation monovalent, alkyle en C₁ à C₈ ou aryle en C₆, phosphato des formes PO₃H₂, PO₃HQ et PO₃Q₂ où Q représente un cation monovalent, alkyle en C₁ à C₈ ou aryle en C₆, tri-(alkyle en C₁ à C₆)silyle, R¹ ou R² pouvant être liés avec l'atome de phosphore adjacent en un cycle aliphatique de 4 à 8 membres qui peut être substitué par alkyle linéaire ou ramifié en C₁ à C₁₀, aryle en C₆, benzyle, alcoxy en C₁ à C₁₀, hydroxy ou benzyloxy, et dans lesquelles
R³-R¹⁴ représentent, indépendamment l'un de l'autre, un atome d'hydrogène ou un radical alkyle en C₁ à C₂₄, halogénoalkyle en C₁ à C₁₀, cycloalkyle en C₃ à C₈, cycloalcényle en C₃ à C₈, le cycle pouvant également contenir 1 ou 2 hétéroatomes choisis dans le groupe N, O, S, aryle en C₆ à C₁₄, phényle, naphtyle, fluorényle, hétéroaryle en C₂ à C₁₃, le nombre d'hétéroatomes choisis dans le groupe N, O, S pouvant être de 1 à 4, les groupes susmentionnés pouvant eux-mêmes être monosubstitués ou polysubstitués, indépendamment l'un de l'autre, par hydrogène, alkyle en C₁ à C₂₀, alcényle en C₂ à C₂₀, halogénoalkyle en C₁ à C₁₀, cycloalkyle en C₃ à C₈, cycloalcényle en C₃ à C₈, hétéroalkyle en C₂ à C₉, hétéroalcényle en C₁ à C₉, aryle en C₆ à C₈, phényle, naphtyle, fluorényle, hétéroaryle en C₂ à C₆, le nombre d'hétéroatomes, en particulier du groupe N, O, S, pouvant être de 1 à 4, alcoxy en C₁ à C₁₀, trihalogénométhylalkyle en C₁ à C₉, trifluorométhyle, trichlorométhyle, fluoro, chloro, bromo, iodo, hydroxy, trifluorométhylsulfonato, oxo, thio, thiolato, amino, amino substitué en C₁ à C₈ des formes NH₂, NH-(alkyle en C₁ à C₈), NH-(aryle en C₅ à C₆), N-(alkyle en C₁ à C₈)₂, N-(aryle en C₅-C₆)₂, N-(alkyle en C₁ à C₈)₃⁺, N-(aryle en C₅-C₆)₃⁺, NH-CO-(alkyle en C₁ à C₈), NH-CO-(aryle en C₅ à C₆), cyano, carboxylato des formes COOH et COOQ, où Q représente un cation monovalent ou un groupe alkyle en C₁ à C₈, acyloxy en C₁ à C₆, sulfinato, sulfonato des formes SO₃H et SO₃Q où Q représente un cation monovalent, alkyle en C₁ à C₈ ou aryle en C₆, phosphato des formes PO₃H₂, PO₃HQ et PO₃Q₂ où Q représente un cation monovalent, alkyle en C₁ à C₈ ou aryle en C₆, tri-(alkyle en C₁ à C₆)silyle,
et deux de ces substituants pouvant également former un pont, et dans lesquelles
m, n représentent indépendamment l'un de l'autre 1 ou 0, et
P est un phosphore trivalent.

2. Composé selon la revendication 1, **caractérisé en ce que** le radical hétéroaromatique à six membres est un radical pyridyle ou le radical hétéroaromatique à cinq membres est un radical furyle, thiophényle ou pyrrolyle et/ou le système cyclique cycloaliphatique est un radical cyclohexyle, cyclopentyle ou, en tant que partie d'un système annelé, un radical indényle et tétrahydronaphtalinyle ou le système cyclique hétéroaliphatique est un radical tétrahydrofuranyle, tétrahydrothiophényle, pyrrolidinyle, pipéridinyle.

3. Composés selon la revendication 1 ou 2, **caractérisés en ce que** les substituants R¹ et R² représentent, indépendamment l'un de l'autre, un radical 1-méthyléthyle, cyclohexyle, cyclopentyle, phényle, 2-méthylphényle, 3,5-diméthylphényle, 4-méthylphényle, 4-méthoxyphényle, 3,5-bis-(trifluorométhyl)phényle, 4-trifluorométhylphényle, 3,5-diméthyl-4-méthoxyphényle, 4-phénoxyle, 4-dialkyamino, 2-alkylphényle, 3-alkylphényle, 4-alkylphényle, 2,6-dialkylphényle, 3,5-dialkylphényle, 3,4,5-trialkylphényle, 2-alcoxyphényle, 3-alcoxyphényle, 4-alcoxyphényle, 2,6-dialcoxyphényle, 3,5-dialcoxyphényle, 3,4,5-trialcoxyphényle, 3,5-dialkyl-4-alcoxyphényle, 3,5-dialkyl-4-dialkylaminophényle, 4-dialkylamino, 3,5-trifluorométhyle, 4-trifluorométhyle, 2-sulfonyle, 3-sulfonyle, 4-sulfonyle, phényle et naphtyle monohalogéné à tétrahalogéné.

4. Composés selon la revendication 1, **caractérisés en ce que** les composés de formule Ia et Ib sont enrichis en énantiomères.

5. Composés selon la revendication 4, **caractérisés en ce que** l'enrichissement en énantiomères est supérieur à 90%.

6. Composés selon l'une quelconque des revendications précédentes, présentant une structure 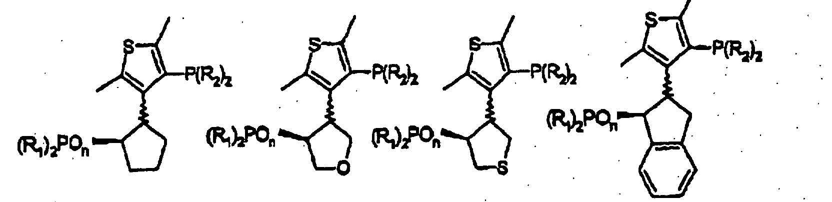 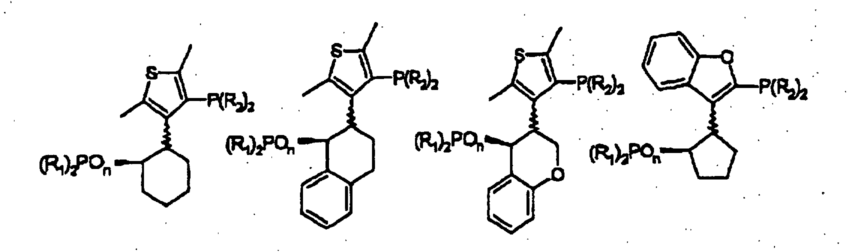 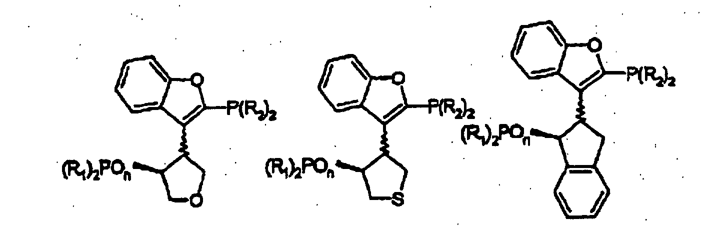 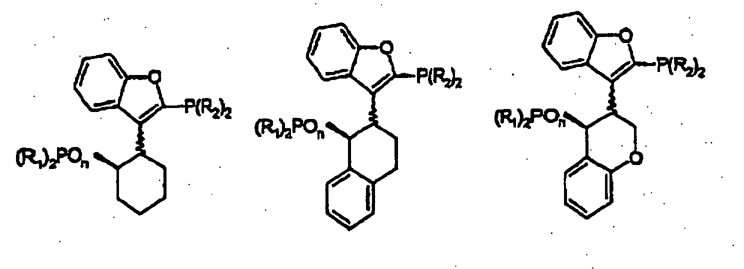 avec n = 0, 1
avec X1, X2, X3 = H, OMe, OEt, F, Me, Et, n = 0, 1 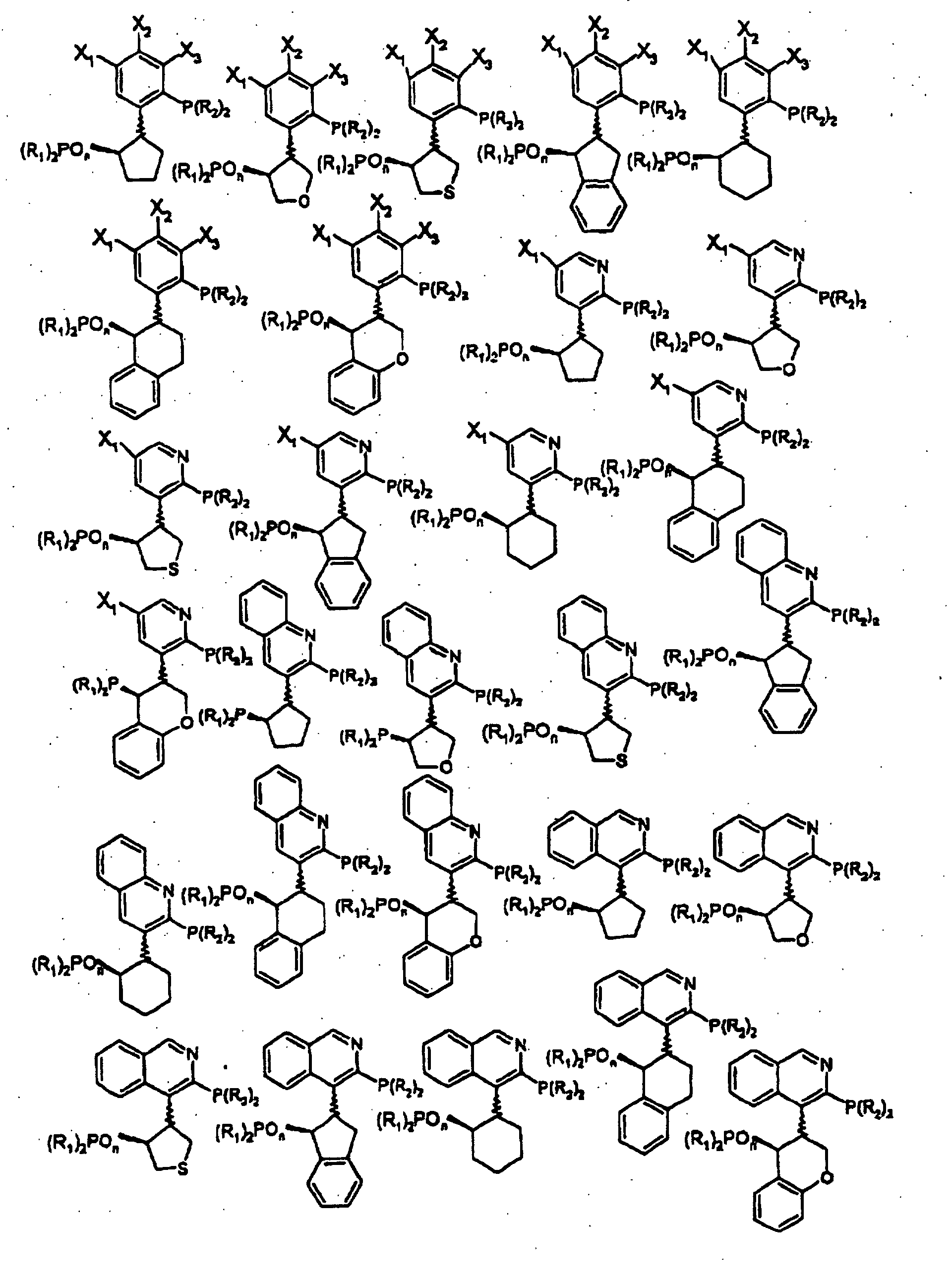 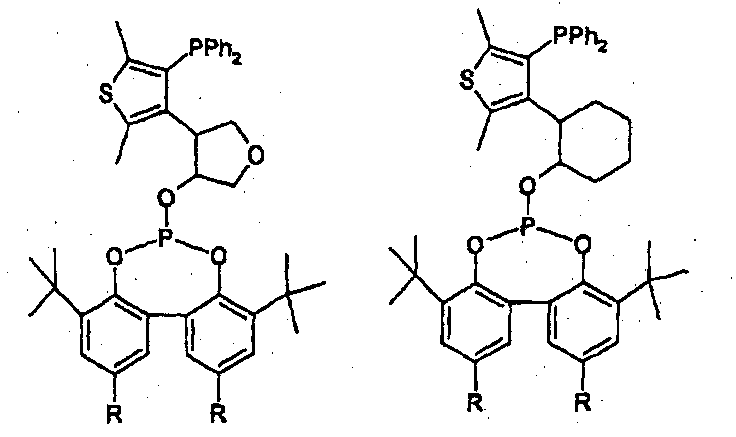 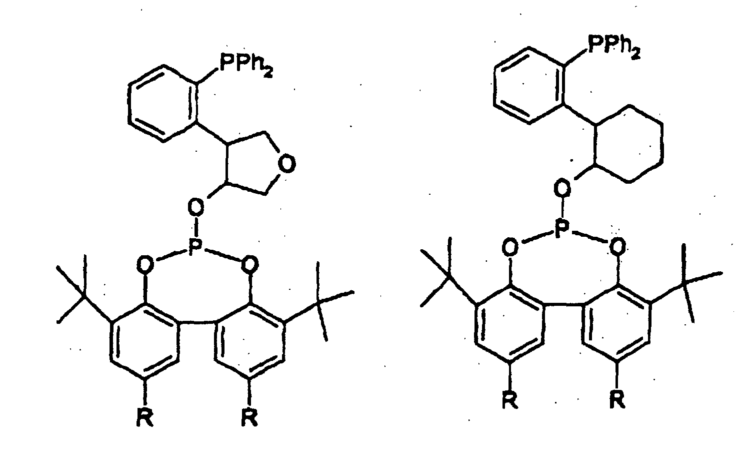 avec R = OMe, t-butyle.

7. Composés complexés de formule générale (II)
[MₓP_{y}L_{z}S_{q}]Aᵣ (II)
où, dans la formule (II)
M est un centre métallique
L représente des ligands organiques ou inorganiques de coordination, identiques ou différents,
S représente des molécules de solvant de coordination et
A représente des équivalents d'anion qui ne sont pas de coordination,
où x et y représentent des nombres entiers supérieurs ou égaux à 1, z, q et r représentent des nombres entiers supérieurs ou égaux à 0, la somme y + z + q est limitée vers le haut par les centres de coordination à disposition sur les centres métalliques, tous les sites de coordination ne devant pas être occupés,
**caractérisés en ce que** P est un ligand organophosphoré bidentate selon l'invention des formules (Ia) et/ou (Ib).

8. Composé complexe qui contient des composés selon la revendication 7, **caractérisé en ce que** les composés contiennent du palladium, du platine, du rhodium, du ruthénium, de l'osmium, de l'iridium, du cobalt, du nickel et/ou du cuivre.

9. Utilisation d'un composé complexe selon la revendication 7 ou 8 comme catalyseur.

10. Procédé pour la préparation de composés des formules (Ia) et (Ib), comprenant les étapes de procédé suivantes :
a) synthèse de la structure de base aliphatique-aromatique par un couplage croisé de Negishi d'iodures de vinyle cycliques avec des halogénoaromatiques ou par ouverture de cycle nucléophile d'un méso-époxyde avec dissociation consécutive des racémates,
b) introduction d'une unité phosphine chirale dans la structure de base par hydroboration asymétrique avec un borane chiral, phosphination avec conservation ou inversion du centre stéréogène et
c) introduction d'un deuxième groupe phosphine par échange brome/lithium avec une base de lithium forte et addition consécutive d'un chlorophosphane.

11. Procédé pour la préparation de biphosphines selon la revendication 10, **caractérisé en ce que** pour la phosphination avec conservation du centre stéréogène, la charge d'hydroboration est soumise à une transmétallisation avant la phosphination avec des diorganylzinciques.

12. Procédé pour la préparation de biphosphines selon la revendication 10, **caractérisé en ce que** pour la phosphination avec inversion du centre stéréogène, la charge d'hydroboration est traitée par oxydation avec formation de l'alcool chiral et le groupe hydroxy chiral est ensuite protégé, en transformant, après la phosphination selon la revendication 10 étape c), le groupe hydroxy par dissociation du groupe de protection en alcool de phosphine et en phosphinant ensuite dans des conditions S_{N}2.

13. Procédé pour la préparation de phosphine-phosphinites et de phosphine-phosphites selon la revendication 10, **caractérisé en ce que** pour la phosphination avec conservation du centre stéréogène, la charge d'hydroboration est traitée par oxydation avec formation de l'alcool chiral, en phosphinant ensuite en présence d'une base.

14. Procédé pour la préparation de phosphine-phosphinites et de phosphine-phosphites selon la revendication 13, **caractérisé en ce que** pour la phosphination avec inversion du centre stéréogène, la charge d'hydroboration est traitée par oxydation avec formation de l'alcool chiral et l'inversion du centre stéréogène est réalisée par une réaction de Mitsonubu ou dans un procédé à deux étapes via une oxydation avec réduction diastéréosélective consécutive.

15. Utilisation d'un composé complexe selon l'une quelconque des revendications 7 à 9 comme catalyseur pour des réactions asymétriques.

16. Utilisation d'un des composés complexes selon l'une quelconque des revendications 7 à 9 comme catalyseur pour les hydrogénations asymétriques, les hydroformylations, les transpositions, les alkylations allyliques, la cyclopropénation, les hydrosilylations, les hydrocyanurations ou les réactions d'aldolisation.
